# EUROPEAN PATENT APPLICATION

(11) **EP 1 616 874 A1**
(43) Date of publication of application: **18.01.2006**
(21) Application number: 04447176.1
(22) Date of filing: 14.07.2004
(51) Int. Cl.: C07H 15/207, A61K 31/7034, A61P 35/00, C07H 15/26

(54) **Mono- and bicyclic compounds with therapeutic activity and pharmaceutical compositions including them**

(71) Applicant: UNIVERSITEIT GENT, 9000 Gent (BE)
(72) Inventor: Van der Eycken, Johan, B-9400 Ninove (BE)
(74) Representative: Bird, William Edward

(57) **Abstract**

This invention relates to novel biologically active monocyclic and polycyclic compounds, in particular bicyclic compounds having some degree of structural similarity with the simmondsin scaffold, as well as monocyclic intermediates for making them. The invention also provides methods for making the novel active compounds and pharmaceutical compositions comprising them.

## Description

### Field of the invention

The invention relates to new monocyclic and bicyclic compounds useful, among other applications, as medicines or for the manufacture of medicaments. The invention also relates to methods of treatment of diseases or functional disorders related to angiogenesis in mammals by the administration of a pharmaceutical composition comprising one or more such monocyclic and bicyclic compounds as biologically active components in combination with one or more pharmaceutically acceptable excipients.

### Background of the invention

In the field of drug discovery there is a constant need for novel scaffolds that enable the rational design of potentially bioactive molecules. Carbohydrates have recently come under scrutiny as offering a source of scaffolds that allow for a high degree of substitution, and offer access to both functional and structural diversity. The nature of saccharide molecules is such that there are numerous different stereoisomers available that can provide access to a greater degree of molecular space than do the scaffolds presently employed in drug discovery.

Simmondsin was first isolated from seeds of the jojoba plant, *Simmondsia californica*, and was reported to exhibit feeding inhibitory activity in animals. The structural study of simmondsin showed that it consists of D-glucose bonded to a substituted cyclohexane derivative bearing an exocyclic α,β-unsaturated nitrile group. Ogawa et al. in J. *Chem. Soc. Perkin Trans* (1992) 1131-1137 published the absolute configuration of simmondsin and its full synthesis while using L-quebrachitol as a starting material for the synthesis of its aglycone moiety.

Starting from the knowledge of a first reported biological activity for naturally occurring simmondsin, there is a need in the art for developing fully synthetic analogues or derivatives of simmondsin in view of exploring their potential biological activities and in view of securing significant amounts thereof independently from the availability of the naturally occurring simmondsin.

### Summary of the Invention

It is an object of the present invention to provide novel biologically active monocyclic and polycyclic compounds, in particular bicyclic compounds having some degree of structural similarity with the simmondsin scaffold. It is another object of the invention to provide novel monocyclic compounds that may be used as intermediates for making the above-referred biologically active monocyclic and polycyclic compounds, in particular the simmondsin-like bicyclic compounds. It is another object of the invention to provide chemical synthetic methods for making said novel monocyclic, bicyclic and polycyclic compounds, either starting from the naturally occurring simmondsin or simmondsin derivatives or by a totally synthetic route. It is yet another object of the invention to provide pharmaceutical compositions based on said novel monocyclic, bicyclic or polycyclic compounds as the biologically active ingredient. It is yet another object of the invention to provide methods of treatment for diseases, in particular angiogenesis-related disorders, in humans by the administration of said pharmaceutical compositions.

The present invention is based on the first unexpected finding that a significant number of novel bicyclic compounds having some degree of structural similarity with the simmondsin scaffold, as well as novel monocyclic compounds (e.g. intermediates therefor) and polycyclic compounds may be obtained by methods including a series of chemical reactions of a known type starting from readily available materials. The present invention is also based on a second unexpected finding that a significant number of novel bicyclic compounds having some degree of structural similarity with the simmondsin scaffold may be obtained by one or more chemical modifications of active substances that may be isolated, using procedures known in the art such as extraction, from the jojoba plant and other simmondsin-containing plants, and in particular simmondsin, derivatives, salts, solvates, stereoisomers or enantiomers thereof. The present invention is also based on a third unexpected finding that a significant number of these novel monocyclic, bicyclic and polycyclic compounds have one or more useful biological activities, in particular a potent angiogenesis-inhibiting activity, an endothelial cell proliferation inhibiting activity and a feeding inhibitory activity in animals, while showing low cytotoxicity *in vitro* and the absence of oestrogen-like activity. The present invention also provides several biological methods and assays for *in vitro* and *in vivo* testing such activities in the novel monocyclic, bicyclic and polycyclic compounds made according to the chemical modifications and synthetic routes set forth.

As a consequence of these biological activities, these novel monocyclic, bicyclic and polycyclic compounds may advantageously be formulated into various kinds of pharmaceutical compositions, especially for the treatment of angiogenesis-related diseases, including arthritis and various types of cancers.

### Definitions

As used herein with respect to a substituting radical, and unless otherwise stated, the term " C₁₋₂₀ alkyl " means straight and branched chain saturated acyclic hydrocarbon monovalent radicals having from 1 to 20 carbon atoms such as, for example, methyl, ethyl, propyl, n-butyl, 1-methylethyl (isopropyl), 2-methylpropyl (isobutyl), 1,1-dimethylethyl (ter-butyl), 2-methylbutyl, n-pentyl, dimethylpropyl, n-hexyl, 2-methylpentyl, 3-methylpentyl, n-heptyl and the like. Furthermore, each of these radicals can be optionally substituted with one or more substituents independently selected from the group consisting of cyano, halogen, azido, oxo, hydroxyl, amino, imino, hydrazino, oximino, carboxyl, C₃₋₁₀ cycloalkyl, aryl, heteroaryl, arylalkyl, C₁₋₂₀ alkoxy, C₁₋₂₀ alkenyloxy, C₁₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, alkylamino, cycloalkylamino, alkenylamino, alkynylamino, cycloalkenylamino, arylamino, arylalkylamino, heterocyclic amino, hydroxyalkylamino, mercaptoalkylamino, alkynylamino, acylamino, thioacylamino, (thio) carboxylic acid (thio)ester and (thio)carboxylic acid amide.

As used herein with respect to a substituting radical, and unless otherwise stated, the term " acyl " broadly refers to a carbonyl (oxo) group adjacent to a C₁₋₂₀ alkyl radical, a C₃₋₁₀ cycloalkyl radical, an aryl radical, an arylalkyl radical or a heterocyclic radical, all of them being such as herein defined; representative examples include acetyl, benzoyl, naphthoyl and the like; similarly, the term " thioacyl " refers to a C=S (thioxo) group adjacent to one of the said radicals.

As used herein with respect to a substituting radical, and unless otherwise stated, the term " C₁₋₂₀ alkylene " means the divalent hydrocarbon radicals corresponding to the above defined C₁₋₂₀ alkyl groups, such as methylene, bis(methylene), tris(methylene), tetramethylene, hexamethylene and the like, including such groups wherein hydrogen atoms are replaced by C₁₋₂₀ alkyl, C₂₋₂₀ alkenyl or C₂₋₂₀ alkynyl groups such as defined herein. Furthermore, each of these radicals can be optionally substituted with one or more substituents independently selected from the group consisting of cyano, halogen, azido, oxo, hydroxyl, amino, imino, hydrazino, oximino, carboxyl, C₃₋₁₀ cycloalkyl, aryl, heteroaryl, arylalkyl, C₁₋₂₀ alkoxy, C₁₋₂₀ alkenyloxy, C₁₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, alkylamino, cycloalkylamino, alkenylamino, alkynylamino, cycloalkenylamino, arylamino, arylalkylamino, heterocyclic amino, hydroxyalkylamino, mercaptoalkylamino, alkynylamino, acylamino, thioacylamino, (thio) carboxylic acid (thio)ester and (thio)carboxylic acid amide.

As used herein with respect to a substituting radical, and unless otherwise stated, the terms " alkylidene ", " cycloalkylidene ", " arylidene ", "heteroarylidene ", refer to divalent hydrocarbon radicals represented by the general formula R'CH=, wherein R' is respectively C₁₋₂₀ alkyl, C₃₋₁₀ cycloalkyl, aryl or heteroaryl (each being as defined herein).

As used herein with respect to a substituting radical, and unless otherwise stated, the term " C₃₋₁₀ cycloalkyl " means a mono- or polycyclic saturated hydrocarbon monovalent radical having from 3 to 10 carbon atoms, such as for instance cyclopropyl, cyclobutyl, cyclopentyl, cyclohexyl, cycloheptyl, cyclooctyl and the like, or a C₇₋₁₀ polycyclic saturated hydrocarbon monovalent radical having from 7 to 10 carbon atoms such as, for instance, norbornyl, fenchyl, trimethyltricycloheptyl or adamantyl, including such groups wherein one or more hydrogen atoms are replaced by C₁₋₂₀ alkyl, C₂₋₂₀ alkenyl or C₂₋₂₀ alkynyl groups such as defined herein. Furthermore, each of these radicals can be optionally substituted with one or more substituents independently selected from the group consisting of cyano, halogen, azido, oxo, hydroxyl, amino, imino, hydrazino, oximino, carboxyl, C₃₋₁₀ cycloalkyl, aryl, heteroaryl, arylalkyl, C₁₋₂₀ alkoxy, C₁₋₂₀ alkenyloxy, C₁₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, alkylamino, cycloalkylamino, alkenylamino, alkynylamino, cycloalkenylamino, arylamino, arylalkylamino, heterocyclic amino, hydroxyalkylamino, mercaptoalkylamino, alkynylamino, acylamino, thioacylamino, (thio) carboxylic acid (thio)ester and (thio)carboxylic acid amide.

As used herein with respect to a substituting radical, and unless otherwise stated, the term " C₃₋₁₀ cycloalkyl-alkyl " refers to an aliphatic saturated hydrocarbon monovalent radical (preferably a C₁₋₂₀ alkyl such as defined above) to which a C₃₋₁₀ cycloalkyl (such as defined above) is already linked such as, but not limited to, cyclohexylmethyl, cyclopentylmethyl and the like.

As used herein with respect to a substituting radical, and unless otherwise stated, the term " N-acyl substituted alkyl " refers to a carbonyl group linked to a divalent aliphatic radical (preferably a C₁₋₂₀ alkylene such as above defined) on one side and to the nitrogen atom of an N-containing group (such as alkylamino, arylamino or heterocyclic amino) on the other side.

As used herein with respect to a substituting radical, and unless otherwise stated, the term " C₃₋₁₀ cycloalkylene " means the divalent hydrocarbon radical corresponding to the above defined C₃₋₁₀ cycloalkyl, e.g. 1,2-cyclohexylene or 1,4-cyclohexylene.

As used herein with respect to a substituting radical, and unless otherwise stated, the term " aryl " designate any mono- or polycyclic aromatic monovalent hydrocarbon radical having from 6 to 30 carbon atoms such as but not limited to phenyl, naphthyl, anthracenyl, phenantracyl, fluoranthenyl, chrysenyl, pyrenyl, biphenylyl, terphenyl, picenyl, indenyl, biphenyl, indacenyl, benzocyclobutenyl, benzocyclooctenyl and the like, including fused benzoC₄₋₈ cycloalkyl radicals (the latter being as defined above) such as, for instance, indanyl, tetrahydronaphtyl, fluorenyl and the like, each of said radicals being optionally substituted with one or more substituents independently selected from the group consisting of halogen, amino, trifluoromethyl, hydroxyl, sulfhydryl and nitro, such as for instance 4-fluorophenyl, 4-chlorophenyl, 3,4-dichlorophenyl, 4-cyanophenyl, 2,6-dichlorophenyl, 2-fluorophenyl, 3-chlorophenyl, 3,5-dichlorophenyl and the like.

As used herein, e.g. with respect to a substituting radical such as a combination of substituents, and unless otherwise stated, the term "homocyclic" means a mono- or polycyclic, saturated or mono-unsaturated or polyunsaturated hydrocarbon radical having from 4 up to 15 carbon atoms but including no heteroatom in the said ring; for instance said may form a C₂₋₆ alkylene radical, such as tetramethylene, which cyclizes with the neighbouring carbon atoms.

As used herein with respect to a substituting radical (including a combination of substituents), and unless otherwise stated, the term " heterocyclic " means a mono- or polycyclic, saturated or mono-unsaturated or polyunsaturated monovalent hydrocarbon radical having from 2 up to 15 carbon atoms and including one or more heteroatoms in one or more heterocyclic rings, each of said rings having from 3 to 10 atoms (and optionally further including one or more heteroatoms attached to one or more carbon atoms of said ring, for instance in the form of a carbonyl or thiocarbonyl or selenocarbonyl group, and/or to one or more heteroatoms of said ring, for instance in the form of a sulfone, sulfoxide, N-oxide, phosphate, phosphonate or selenium oxide group), each of said heteroatoms being independently selected from the group consisting of nitrogen, oxygen, sulfur, selenium and phosphorus, also including radicals wherein a heterocyclic ring is fused to one or more aromatic hydrocarbon rings for instance in the form of benzo-fused, dibenzo-fused and naphto-fused heterocyclic radicals; within this definition are included heterocyclic radicals such as, but not limited to, diazepinyl, oxadiazinyl, thiadiazinyl, dithiazinyl, triazolonyl, diazepinonyl, triazepinyl, triazepinonyl, tetrazepinonyl, benzo-quinolinyl, benzothiazinyl, benzothiazinonyl, benzoxa-thiinyl, benzodioxinyl, benzodithiinyl, benzoxazepinyl, benzothiazepinyl, benzodiazepinyl, benzodioxepinyl, benzodithiepinyl, benzoxazocinyl, benzothiazocinyl, benzodiazocinyl, benzoxathiocinyl, benzodioxocinyl, benzotrioxepinyl, benzoxathiazepinyl, benzoxadiazepinyl, benzothiadiazepinyl, benzotriazepinyl, benzoxathiepinyl, benzotriazinonyl, benzoxazolinonyl, azetidinonyl, azaspiroundecyl, dithiaspirodecyl, selenazinyl, selenazolyl, selenophenyl, hypoxanthinyl, azahypoxanthinyl, bipyrazinyl, bipyridinyl, oxazolidinyl, diselenopyrimidinyl, benzodioxocinyl, benzopyrenyl, benzo-pyranonyl, benzophenazinyl, benzoquinolizinyl, dibenzocarbazolyl, dibenzoacridinyl, dibenzophenazinyl, dibenzothiepinyl, dibenzooxepinyl, dibenzopyranonyl, dibenzoquinoxalinyl, dibenzothiazepinyl, dibenzoiso-quinolinyl, tetraazaadamantyl, thiatetraazaadamantyl, oxauracil, oxazinyl, dibenzothiophenyl, dibenzofuranyl, oxazolinyl, oxazolonyl, azaindolyl, azolonyl, thiazolinyl, thiazolonyl, thiazolidinyl, thiazanyl, pyrimidonyl, thiopyrimidonyl, thiamorpho-linyl, azlactonyl, naphtindazolyl, naphtindolyl, naphtothiazolyl, naphtothioxolyl, naphtoxindolyl, naphtotriazolyl, naphto-pyranyl, oxabicycloheptyl, azabenz-imidazolyl, azacycloheptyl, azacyclooctyl, azacyclononyl, azabicyclononyl, tetrahydrofuryl, tetrahydropyranyl, tetrahydropyronyl, tetrahydroquinoleinyl, tetrahydrothienyl and dioxide thereof, dihydrothienyl dioxide, dioxindolyl, dioxinyl, dioxenyl, dioxazinyl, thioxanyl, thioxolyl, thiourazolyl, thiotriazolyl, thiopyranyl, thiopyronyl, coumarinyl, quinoleinyl, oxyquinoleinyl, quinuclidinyl, xanthinyl, dihydropyranyl, benzodihydrofuryl, benzothiopyronyl, benzothio-pyranyl, benzoxazinyl, benzoxazolyl, benzodioxolyl, benzodioxanyl, benzothiadiazolyl, benzotriazinyl, benzothiazolyl, benzoxazolyl, phenothioxinyl, phenothiazolyl, phenothienyl (benzothiofuranyl), phenopyronyl, phenoxazolyl, pyridinyl, dihydropyridinyl, tetrahydropyridinyl, piperidinyl, morpholinyl, thiomorpholinyl, pyrazinyl, pyrimidinyl, pyridazinyl, triazinyl, tetrazinyl, triazolyl, benzotriazolyl, tetrazolyl, imidazolyl, pyrazolyl, thiazolyl, thiadiazolyl, isothiazolyl, oxazolyl, oxadiazolyl, pyrrolyl, furyl, dihydrofuryl, furoyl, hydantoinyl, dioxolanyl, dioxolyl, dithianyl, dithienyl, dithiinyl, thienyl, indolyl, indazolyl, benzofuryl, quinolyl, quinazolinyl, quinoxalinyl, carbazolyl, phenoxazinyl, phenothiazinyl, xanthenyl, purinyl, benzothienyl, naphtothienyl, thianthrenyl, pyranyl, pyronyl, benzopyronyl, isobenzofuranyl, chromenyl, phenoxathiinyl, indolizinyl, quinolizinyl, isoquinolyl, phthalazinyl, naphthiridinyl, cinnolinyl, pteridinyl, carbolinyl, acridinyl, perimidinyl, phenanthrolinyl, phenazinyl, phenothiazinyl, imidazolinyl, imidazolidinyl, benzimidazolyl, pyrazolinyl, pyrazolidinyl, pyrrolinyl, pyrrolidinyl, piperazinyl, uridinyl, thymidinyl, cytidinyl, azirinyl, aziridinyl, diazirinyl, diaziridinyl, oxiranyl, oxaziridinyl, dioxiranyl, thiiranyl, azetyl, dihydroazetyl, azetidinyl, oxetyl, oxetanyl, thietyl, thietanyl, diazabicyclooctyl, diazetyl, diaziridinonyl, diaziridinethionyl, chromanyl, chromanonyl, thiochromanyl, thiochromanonyl, thiochromenyl, benzofuranyl, benzisothiazolyl, benzo-carbazolyl, benzochromonyl, benzisoalloxazinyl, benzocoumarinyl, thiocoumarinyl, phenometoxazinyl, phenoparoxazinyl, phentriazinyl, thio-diazinyl, thiodiazolyl, indoxyl, thioindoxyl, benzodiazinyl (e.g. phtalazinyl), phtalidyl, phtalimidinyl, phtalazonyl, alloxazinyl, dibenzopyronyl (i.e. xanthonyl), xanthionyl, isatyl, isopyrazolyl, isopyrazolonyl, urazolyl, urazinyl, uretinyl, uretidinyl, succinyl, succinimido, benzylsultimyl, benzylsultamyl and the like, including all possible isomeric forms thereof, wherein each carbon atom of said heterocyclic ring may be independently substituted with a substituent selected from the group consisting of halogen, nitro, C₁₋₂₀ alkyl, C₂₋₂₀ alkenyl, C₂₋₂₀ alkynyl, halo C₁₋₂₀ alkyl, C₃₋₁₀ cycloalkyl, aryl, arylalkyl, alkylaryl, alkylacyl, arylacyl, hydroxyl, amino, C₁₋₂₀ alkylamino, cycloalkylamino, alkenylamino, cycloalkenylamino, alkynylamino, arylamino, arylalkylamino, hydroxyalkylamino, mercaptoalkylamino, heterocyclic amino, hydrazino, alkylhydrazino, phenylhydrazino, sulfhydryl, C₁₋₂₀ alkoxy, C₃₋₁₀ cycloalkoxy, aryloxy, arylalkyloxy, oxyheterocyclic, heterocyclic-substituted alkyloxy, thio C₁₋₂₀ alkyl, thio C₃₋₁₀ cycloalkyl, thioaryl, thioheterocyclic, arylalkylthio, heterocyclic-substituted alkylthio, formyl, hydroxylamino, cyano, carboxylic acid or esters or thioesters or amides thereof, thiocarboxylic acid or esters or thioesters or amides thereof; depending upon the number of unsaturations in the 3 to 10 membered ring, heterocyclic radicals may be subdivided into heteroaromatic (or "heteroaryl") radicals and non-aromatic heterocyclic radicals; when a heteroatom of the said non-aromatic heterocyclic radical is nitrogen, the latter may be substituted with a substituent selected from the group consisting of C₁₋₇ alkyl, C₃₋₁₀ cycloalkyl, aryl, arylalkyl and alkylaryl.

As used herein with respect to a substituting radical, and unless otherwise stated, the terms " C₁₋₂₀ alkoxy ", " C₃₋₁₀ cycloalkoxy ", " aryloxy", "arylalkyloxy ", " oxyheterocyclic ", " C₁₋₂₀ alkylthio ", " C₃₋₁₀ cycloalkylthio ", "arylthio ", " arylalkylthio " and " thioheterocyclic " refer to substituents wherein a C₁₋₂₀ alkyl radical, respectively a C₃₋₁₀ cycloalkyl, aryl, arylalkyl or heterocyclic radical (each of them such as defined herein), are attached to an oxygen atom or a divalent sulfur atom through a single bond, such as but not limited to methoxy, ethoxy, propoxy, butoxy, pentoxy, isopropoxy, sec-butoxy, tert-butoxy, isopentoxy, cyclopropyloxy, cyclobutyloxy, cyclopentyloxy, thiomethyl, thioethyl, thiopropyl, thiobutyl, thiopentyl, thiocyclopropyl, thiocyclobutyl, thiocyclopentyl, thiophenyl, phenyloxy, benzyloxy, mercaptobenzyl, cresoxy, and the like.

As used herein with respect to a substituting atom, and unless otherwise stated, the term halogen means any atom selected from the group consisting of fluorine, chlorine, bromine and iodine.

As used herein with respect to a substituting radical, and unless otherwise stated, the term " halo C₁₋₂₀ alkyl " means a C₁₋₂₀ alkyl radical (such as above defined) in which one or more hydrogen atoms are independently replaced by one or more halogens (preferably fluorine, chlorine or bromine), such as but not limited to difluoromethyl, trifluoromethyl, trifluoroethyl, octafluoropentyl, dodecafluoroheptyl, dichloromethyl and the like.

As used herein with respect to a substituting radical, and unless otherwise stated, the terms " C₂₋₂₀ alkenyl " designate a straight and branched acyclic hydrocarbon monovalent radical having one or more ethylenic unsaturations and having from 2 to 20 carbon atoms such as, for example, vinyl, 1-propenyl, 2-propenyl (allyl), 1-butenyl, 2-butenyl, 2-pentenyl, 3-pentenyl, 3-methyl-2-butenyl, 3-hexenyl, 2-hexenyl, 2-heptenyl, 1,3-butadienyl, pentadienyl, hexadienyl, heptadienyl, heptatrienyl and the like, including all possible isomers thereof.

As used herein with respect to a substituting radical, and unless otherwise stated, the term " C₃₋₁₀ cycloalkenyl " mean a monocyclic mono- or polyunsaturated hydrocarbon monovalent radical having from 3 to 8 carbon atoms, such as for instance cyclopropenyl, cyclobutenyl, cyclopentenyl, cyclopentadienyl, cyclohexenyl, cyclohexadienyl, cycloheptenyl, cyclohepta-dienyl, cycloheptatrienyl, cyclooctenyl, cyclooctadienyl and the like, or a C₇-₁₀ polycyclic mono- or polyunsaturated hydrocarbon mono-valent radical having from 7 to 10 carbon atoms such as dicyclopentadienyl, fenchenyl (including all isomers thereof, such as α-pinolenyl), bicyclo[2.2.1]hept-2-enyl, bicyclo[2.2.1] hepta-2,5-dienyl, cyclofenchenyl and the like.

As used herein with respect to a substituting radical, and unless otherwise stated, the term " C₂₋₂₀ alkynyl " defines straight and branched chain hydrocarbon radicals containing one or more triple bonds and optionally at least one double bond and having from 2 to 20 carbon atoms such as, for example, acetylenyl, 1-propynyl, 2-propynyl, 1-butynyl, 2-butynyl, 2-pentynyl, 1-pentynyl, 3-methyl-2-butynyl, 3-hexynyl, 2-hexynyl, 1-penten-4-ynyl, 3-penten-1-ynyl, 1,3-hexadien-1-ynyl and the like.

As used herein with respect to a substituting radical, and unless otherwise stated, the terms " arylalkyl ", " arylalkenyl " and "heterocyclic-substituted alkyl " refer to an aliphatic saturated or ethylenically unsaturated hydrocarbon monovalent radical (preferably a C₁₋₂₀ alkyl or C₂₋₂₀ alkenyl radical such as defined above) onto which an aryl or heterocyclic radical (such as defined above) is already bonded, and wherein the said aliphatic radical and/or the said aryl or heterocyclic radical may be optionally substituted with one or more substituents independently selected from the group consisting of C₁₋₄ alkyl, trifluoromethyl, halogen, amino, nitro, hydroxyl, sulfhydryl and nitro, such as but not limited to benzyl, 4-chlorobenzyl, 2-fluorobenzyl, 4-fluorobenzyl, 3,4-dichlorobenzyl, 2,6-dichlorobenzyl, 4-*ter*-butylbenzyl, 3-methylbenzyl, 4-methylbenzyl, phenylpropyl, 1-naphtylmethyl, phenylethyl, 1-amino-2-phenylethyl, 1-amino-2-[4-hydroxy-phenyl]ethyl, 1-amino-2-[indol-2-yl]ethyl, styryl, pyridylmethyl (including all isomers thereof), pyridylethyl, 2-(2-pyridyl)isopropyl, oxazolylbutyl, 2-thienylmethyl, pyrrolylethyl, morpholinyl-ethyl, imidazol-1-yl-ethyl, benzodioxolylmethyl and 2-furylmethyl.

As used herein with respect to a substituting radical, and unless otherwise stated, the term " alkylaryl " and "alkyl-substituted heterocyclic" refer to an aryl or heterocyclic radical (such as defined above) onto which are bonded one or more aliphatic saturated hydrocarbon mono-valent radicals, preferably one or more C₁₋₂₀ alkyl or C₃₋₁₀ cycloalkyl radicals as defined above such as, but not limited to, o-toluyl, m-toluyl, p-toluyl, 2,3-xylyl, 2,4-xylyl, 3,4-xylyl, o-cumenyl, m-cumenyl, p-cumenyl, o-cymenyl, m-cymenyl, p-cymenyl, mesityl, *ter*-butylphenyl, lutidinyl (i.e. dimethylpyridyl), 2-methylaziridinyl, methylbenzimidazolyl, methylbenzofuranyl, methylbenzothiazolyl, methyl-benzotriazolyl, methylbenzoxazolyl and methylbenzselenazolyl.

As used herein with respect to a substituting radical, and unless otherwise stated, the term " alkoxyaryl " refers to an aryl radical (such as defined above) onto which is (are) bonded one or more C₁₋₂₀ alkoxy radicals as defined above, preferably one or more methoxy radicals, such as, but not limited to, 2-methoxyphenyl, 3-methoxyphenyl, 4-methoxyphenyl, 3,4-dimethoxyphenyl, 2,4,6-trimethoxyphenyl, methoxynaphtyl and the like.

As used herein with respect to a substituting radical, and unless otherwise stated, the terms " alkylamino ", "cycloalkylamino ", "alkenyl-amino", " cycloalkenylamino '' , " arylamino ", "arylalkylamino ", "heterocyclic amino " , " hydroxyalkylamino ", "mercaptoalkylamino " and " alkynylamino" mean that respectively one (thus monosubstituted amino) or even two (thus disubstituted amino) C₁₋₂₀ alkyl, C₃₋₁₀ cycloalkyl, C₂₋₂₀ alkenyl, C₃₋₁₀ cycloalkenyl, aryl, arylalkyl, heterocyclic, mono- or polyhydroxy C₁₋₇ alkyl, mono- or polymercapto C₁₋₂₀ alkyl or C₂₋₂₀ alkynyl radical(s) (each of them as defined herein, respectively) is/are attached to a nitrogen atom through a single bond or, in the case of heterocyclic, include a nitrogen atom, such as but not limited to, anilino, benzylamino, methylamino, dimethylamino, ethylamino, diethyl-amino, isopropylamino, propenylamino, n-butylamino, ter-butylamino, dibutylamino, morpholinoalkylamino, morpholinyl, piperidinyl, piperazinyl, hydroxymethylamino, β-hydroxyethylamino and ethynylamino; this definition also includes mixed disubstituted amino radicals wherein the nitrogen atom is attached to two such radicals belonging to two different sub-set of radicals, e.g. an alkyl radical and an alkenyl radical, or to two different radicals within the same sub-set of radicals, e.g. methylethylamino; among disubstituted amino radicals, symetrically substituted are more easily accessible and thus usually preferred.

As used herein with respect to a substituting radical, and unless otherwise stated, the terms "(thio)carboxylic acid ester ", " (thio)carboxylic acid thioester " and " (thio)carboxylic acid amide" refer to radicals wherein a carbonyl or thiocarbonyl group is bonded to an alkoxy, a mercapto, an aryloxy, an arylthio, an amino, a primary or secondary amino, or a hydroxylamino, the residue on the oxygen, nitrogen or sulfur atom thereof being selected from the group consisting of C₁₋₂₀ alkyl, C₂₋₂₀ alkenyl, C₂₋₂₀ alkynyl, C₃₋₁₀ cycloalkyl, cycloalkenyl, cycloheteroalkyl, aryl, heteroaryl, arylalkyl, alkylaryl, all of the latter being such as defined herein, respectively.

As used herein with respect to a substituting radical, and unless otherwise stated, the term " amino-acid " refers to a radical derived from a molecule having the chemical formula H₂N-CHR-COOH, wherein R is the side group of atoms characterizing the amino-acid type; said molecule may be one of the 20 naturally-occurring amino-acids or any similar non naturally-occurring amino-acid, including cyclic amino-acids and acyclic or cyclic β-amino-acids.

As used herein and unless otherwise stated, the term " stereoisomer " refers to all possible different isomeric as well as conformational forms which the compounds of the invention may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

As used herein and unless otherwise stated, the term " enantiomer " means each individual optically active form of a compound of the invention, having an optical purity or enantiomeric excess (as determined by methods standard in the art) of at least 80% (i.e. at least 90% of one enantiomer and at most 10% of the other enantiomer), preferably at least 90% and more preferably at least 98%.

As used herein and unless otherwise stated, the term " solvate " includes any combination which may be formed by a compound of this invention with a suitable inorganic solvent (e.g. hydrates) or organic solvent, such as but not limited to alcohols, ketones, esters, ethers, bases, nitriles, amides, sulfur-containing compounds, halogenated hydrocarbons, aromatic and aliphatic hydrocarbons. Specific examples thereof include, but are not limited to, ethanol, 2-propanol, ethyl acetate, diethyl ether, pyridine, N-methyl pyrrolidone, acetonitrile, dimethylformamide, acetone, methylethylketone, dimethylsulfoxide, dichloromethane, chloroform and toluene.

As used herein and unless otherwise stated, the term " salt " refers to conventional non-toxic salts which may be formed through addition of an inorganic or organic acid or base. Examples of such acid addition salts include, but are not limited to, acetate, adipate, alginate, aspartate, benzoate, benzenesulfonate, sulfate, butyrate, citrate, camphorate, camphor-sulfonate, propionate, digluconate, dodecyl-sulfate, erucate, ethanesulfonate, ferulate, fumarate, glucoheptanoate, glycerophosphate, heptanoate, hexanoate, hydrochloride, hydrobromide, 2-hydroxyethanesulfonate, lactate, maleate, malonate, methanesulfonate, 2-naphthalenesulfonate, nicotinate, oxalate, pamoate, pectinate, 3-phenyl-propionate, picrate, pivalate, propionate, salicylate, succinate, tartrate, thiocyanate, p-toluenesulfonate, undecanoate and valerate. For therapeutic use, said salts are preferably those wherein the counter-ion is pharmaceutically or physiologically acceptable. Examples of such base addition salts include, but are not limited to, ammonium salts, alkali and alkaline-earth metal salts (e. g. lithium, sodium, potassium, magnesium and calcium salts), salts with organic bases such as benzathine, N-methyl-D-glucamine and hydrabamine, or with amino-acids such as, for example, arginine, lysine and the like.

As used herein and unless otherwise stated, the term " alkanoyl " refers to a alkyl radical linked to a carbonyl moiety.

As used herein and unless otherwise stated, the term " aminoalkanoyl " refers to a group derived from an amino-substituted saturated carboxylic acid wherein the amino group may be a primary, secondary or tertiary amino group.

As used herein and unless otherwise stated, the term " aminocarbonyl" refers to an amino-substituted carbonyl (carbamoyl) group wherein the amino group can be a primary, secondary or tertiary amino group.

As used herein and unless otherwise stated, the term " aralkanoyl " refers to a radical derived from an aryl-substituted saturated carboxylic acid such as, but not limited to, phenylacetyl, 3-phenylpropionyl (hydrocinnamoyl), 4-phenylbutyryl, (2-naphthyl)acetyl, 4-chlorohydrocinnamoyl, 4-aminohydro-cinnamoyl and 4- methoxyhydrocinnamoyl.

As used herein and unless otherwise stated, the term "aralkoxycarbonyl " refers to a radical such as, but not limited to, benzyloxycarbonyl and 4-methoxyphenylmethoxycarbonyl.

As used herein and unless otherwise stated, the term " aroyl " refers to a radical derived from an aromatic carboxylic acid such as, but not limited to, benzoyl, 4-chlorobenzoyl, 4- carboxybenzoyl, 4- (benzyloxycarbonyl) benzoyl, 1-naphthoyl, 2-naphthoyl, 6-carboxy-2-naphthoyl, 6-benzyloxycarbonyl-2-naphthoyl, 3-benzyloxy-2-naphthoyl and 3-hydroxy-2-naphthoyl.

As used herein and unless otherwise stated, the term "cycloalkylcarbonyl " refers to a group derived from a monocyclic or bridged cycloaliphatic carboxylic acid such as, but not limited to, cyclopropylcarbonyl, cyclohexylcarbonyl, adamantylcarbonyl, or from a benzo-fused monocyclic cycloaliphatic carboxylic acid such as, but not limited to, 1,2,3,4-tetrahydro-2-naphthoyl and 2-acetamido-1,2,3,4-tetrahydro-2-naphthoyl.

The terms " angiogenesis " and " neovascularisation ", as used herein, refer to the phenomenon of new blood vessel formation. Blood vessel formation is required for normal tissue growth, placenta and embryonic development, wound healing and the like.

The term " angiogenesis-inhibiting ", as used herein, refers to the ability to at least partly inhibit, prevent, or greatly reduce the formation or outgrowth of blood or lymph vessels, or the ability to destroy such vessels during sprouting or outgrowth *in vitro* as well as *in vivo.*

The term " angiogenesis-related disease ", as used herein, refers to diseases wherein angiogenesis plays a crucial detrimental role such as, but not limited to, cancer, hemangiomas, atherosclerosis, inflammatory diseases, arthritis, psoriasis, pre-eclampsia, intra-uterine growth retardation, endometriosis, liver or kidney fibrosis, proliferative retinopathy, age-related maculopathy, diabetes mellitus related maculopathy, diabetic retinopathy, macular degeneration , neovascular glaucoma, retrolental fibroplasias, retinal vascularisation and the like.

The term " arthritis " as used herein, refers to any form of joint inflammation and includes, but is not limited to, osteoarthritis, rheumatoid arthritis, ankylosing spondylitis, bursitis, fibromyalgia, gout, infectious arthritis, juvenile rheumatoid arthritis, psoriatic arthritis, reactive arthritis, scleroderma, systemic lupus erythematosus, tendonitis and synovitis.

The term " therapeutically effective amount ", as used herein, means an amount that elicits a measurable biological response in a tissue, organ or system of an animal, preferably a human, or which alleviates the symptoms of a disease being treated.

### Detailed description of the invention

More specifically, the present invention relates to a class of novel monocyclic, bicyclic and polycyclic compounds having the general formula **1**

X-d-L-e-Y

wherein:
- X is a moiety represented by the general formula **2** shown in claim 1 wherein:
   - A and B are each independently selected from the group consisting of hydrogen, cyano, halogen, azido, C=NOR₆, C=N-NR₇R₈, COOR₆, CONR₇R₈, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, arylalkyl, alkoxyaryl and heterocyclic, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, C₁₋₂₀ alkylamino, C₃₋₁₀ cycloalkylamino, C₂₋₂₀ alkenylamino, C₃₋₁₀ cycloalkenylamino, arylamino, arylalkylamino, heterocyclic amino, hydroxyalkylamino, mercaptoalkylamino and C₂₋₂₀ alkynylamino, acylamino, thioacylamino, or A and B together form a homocyclic or heterocyclic group, provided that A and B are not both hydrogen,
   - T₁, T₂, T₃, T₄ and T₅ are carbon atoms, or alternatively one of T₁, T₂, T₃, T₄ and T₅ is oxygen or nitrogen while the remaining of T₁, T₂, T₃, T₄ and T₅ are carbon atoms, or alternatively two of T₁, T₂, T₃, T₄ and T₅ are either both nitrogen, or oxygen and nitrogen, while the remaining of T₁, T₂, T₃, T₄ and T₅ are carbon atoms, or alternatively two of non adjacent T₁, T₂, T₃, T₄ and T₅ are oxygen while the remaining of T₁, T₂, T₃, T₄ and T₅ are carbon atoms,
   - R₁, R₂, R₃, R₄ and R₅ are each substituents independently selected from the group consisting of hydrogen, cyano, halogen, azido, hydroxyl, amino, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, (di)C₁₋₂₀ alkylamino(thio)carbonyloxy, C₃₋₁₀ cycloalkylaminocarbonyloxy, C₁₋₂₀ alkylthio, C₃₋₁₀ cycloalkylthio, arylthio, arylalkylthio, acylthio, thioheterocyclic, C₁₋₂₀ alkylamino, C₃₋₁₀ cycloalkylamino, C₂₋₂₀ alkenyl-amino, C₃₋₁₀ cycloalkenylamino, arylamino, arylalkylamino, heterocyclic amino, hydroxyalkylamino, mercaptoalkylamino, C₂₋₂₀ alkynylamino, acylamino, thioacylamino, (hetero)aroylamino, (di)C₁₋₂₀ alkylamino(thio) carbonylamino, C₁₋₂₀ alkoxy(thio)carbonylamino, (thio) carboxylic acid (thio)ester and (thio)carboxylic acid amide, with the first proviso that if any one of T₁, T₂, T₃, T₄ and T₅ is oxygen then the correspondingly borne substituent R₁, R₂, R₃, R₄ or R₅ is absent, and with the second proviso that if any one of T₁, T₂, T₃, T₄ and T₅ is nitrogen then the correspondingly borne substituent R₁, R₂, R₃, R₄ or R₅ cannot be cyano, halogen, azido, carboxyl, C₁₋₂₀ alkylthio, C₃₋₁₀ cycloalkylthio, arylthio, arylalkylthio, acylthio or thioheterocyclic,
   - S₁, S₂, S₃, S₄ and S₅ are each substituents independently selected from the group consisting of hydrogen, cyano, halogen, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkyl-thio, acylthio, thioheterocyclic, (thio) carboxylic acid (thio)ester and (thio)carboxylic acid amide, with the proviso that if any one of T₁, T₂, T₃, T₄ and T₅ is oxygen or nitrogen then the correspondingly borne substituent S₁, S₂, S₃, S₄ and S₅ is absent,
   - if any one of T₁, T₂, T₃, T₄ and T₅ is a carbon atom then each pair of geminal substituents borne by the same carbon atom, i.e. (R₁,S₁), (R₂,S₂), (R₃,S₃), (R₄,S₄) or (R₅,S₅), may independently form a homocyclic or heterocyclic ring, or an alkylidene, cycloalkylidene or arylalkylidene group, or a carbonyl (oxo) or thiocarbonyl (thioxo) group, or an imino, an oximino or a hydrazone group,
   - R₆ is selected from the group consisting of hydrogen, C₁₋₂₀ alkyl, C₂₋₂₀ alkenyl, C₂₋₂₀ alkynyl, C₃₋₁₀ cycloalkyl, C₃₋₁₀ cycloalkenyl, aryl, arylalkyl and heteroaryl,
   - R₇ and R₈ are each independently selected from the group consisting of hydrogen, C₁₋₂₀ alkyl, C₂₋₂₀ alkenyl, C₂₋₂₀ alkynyl, C₃₋₁₀ cycloalkyl, C₃₋₁₀ cycloalkenyl, aryl, arylalkyl and heteroaryl, or R₇ and R₈ together may form a homocyclic or heterocyclic group, and
   - m and n are each integers independently selected from 0, 1 and 2,
- Y is an amino-acid residue or a moiety represented by the general formula **3** shown in claim 1 wherein:
   - U₁, U₂, U₃, U₄ and U₅ are carbon atoms, or alternatively one of U₁, U₂, U₃, U₄, and U₅ is oxygen or nitrogen while the remaining of U₁, U₂, U₃, U₄ and U₅ are carbon atoms, or alternatively two of U₁, U₂, U₃, U₄ and U₅ are either both nitrogen, or oxygen and nitrogen, while the remaining of U₁, U₂, U₃, U₄ and U₅ are carbon atoms, or alternatively two of non adjacent U₁, U₂, U₃, U₄ and U₅ are oxygen while the remaining of U₁, U₂, U₃, U₄ and U₅ are carbon atoms,
   - P₁, P₂, P₃, P₄ and P₅ are each substituents independently selected from the group consisting of hydrogen, cyano, halogen, azido, hydroxyl, amino, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, (di) C₁₋₂₀ alkylamino(thio)carbonyloxy, C₁₋₂₀ alkylthio, C₃₋₁₀ cycloalkylthio, arylthio, arylalkylthio, acylthio, thio-heterocyclic, C₁₋₂₀ alkylamino, C₃₋₁₀ cycloalkylamino, C₂₋₂₀ alkenyl-amino, C₃₋₁₀ cycloalkenylamino, arylamino, arylalkylamino, heterocyclic amino, hydroxyalkylamino, mercaptoalkylamino, C₂₋₂₀ alkynylamino, acylamino, thioacylamino, (hetero)aroylamino, (di)C₁₋₂₀ alkylamino(thio)carbonylamino, C₁₋₂₀ alkoxy(thio)carbonylamino, (thio) carboxylic acid (thio)ester and (thio)carboxylic acid amide, with the first proviso that if any one of U₁, U₂, U₃, U₄ and U₅ is oxygen then the correspondingly borne substituent P₁, P₂, P₃, P₄ or P₅ is absent, and with the second proviso that if any one of U₁, U₂, U₃, U₄ and U₅ is nitrogen then the correspondingly borne substituent P₁, P₂, P₃, P₄ or P₅ cannot be cyano, halogen, azido, carboxyl, C₁₋₂₀ alkylthio, C₃₋₁₀ cycloalkylthio, arylthio, arylalkylthio, acylthio or thioheterocyclic,
   - Q₁, Q₂, Q₃, Q₄ and Q₅ are each substituents independently selected from the group consisting of hydrogen, cyano, halogen, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, (thio) carboxylic acid (thio)ester and (thio)carboxylic acid amide, with the proviso that if any one of U₁, U₂, U₃, U₄ and U₅ is oxygen or nitrogen then the correspondingly borne substituent Q₁, Q₂, Q₃, Q₄ or Q₅ is absent,
   - if any one of U₁, U₂, U₃, U₄ and U₅ is a carbon atom then a pair of geminal substituents borne by the same carbon atom, i.e. (P₁,Q₁), (P₂,Q₂), (P₃,Q₃), (P₄,Q₄) or (P₅,Q₅) may independently form a homocyclic or heterocyclic ring, or an alkylidene, cycloalkylidene or arylalkylidene group, or a carbonyl (oxo) or thiocarbonyl (thioxo) group, or an imino, an oximino or a hydrazone group,
- L is a linker selected from the group consisting of a covalent bond, C₁₋₂₀ alkyl, C₃₋₁₀ cycloalkyl, C₂₋₂₀ alkenyl, C₃₋₁₀ cycloalkenyl, C₂₋₂₀ alkynyl, heteroalkyl, cycloheteroalkyl, aryl, heteroaryl, arylalkyl and heteroarylalkyl,
- d represents a moiety for the attachment of X and L, which replaces any one of the substituents R₁, R₂, R₃, R₄, R₅, S₁, S₂, S₃, S₄ and S₅,
- e represents a moiety for the attachment of Y and L, which replaces any one of the substituents P₁, P₂, P₃, P₄, P₅, Q₁, Q₂, Qs, Q₄ and Q₅, and
- each of d and e is a covalent bond, an oxygen atom, a sulfur atom or a divalent radical selected from the group consisting of -N(Z)-, -(C=O)N(Z)-, - SO₂N(Z)-, -S(=O)N(Z)-, -OP(=O)(OW)N(Z)-, -N(Z)C(=O)N(Z)-, -N(Z)C(=O)O-, -N(Z)C(=S)N(Z)-, -N(Z)C(=S)O-, -C(=O)O-, -N(Z)C(=S)Sand -C(=O)-,
- Z is selected from the group consisting of hydrogen, C₁₋₂₀ alkyl, C₂₋₂₀ alkenyl, C₂₋₂₀ alkynyl, acyl, aroyl, heteroaroyl, aryl, heteroaryl, arylalkyl and heteroarylalkyl, and
- W is selected from the group consisting of hydrogen, C₁₋₂₀ alkyl, C₂₋₂₀ alkenyl, C₂₋₂₀ alkynyl, aryl, heteroaryl, arylalkyl and heteroarylalkyl,
a salt, a solvate, a stereoisomer or an enantiomer thereof.

The present invention also relates to monocyclic compounds having the general formula **2** shown in claim 1 with all substituents and moieties being as defined herein-above.

In another specific embodiment, the invention relates to a sub-class of such monocyclic, bicyclic and polycyclic compounds having the general formula **1**, as well as a sub-class of such monocyclic compounds having the general formula **2**, wherein m is 1 and wherein none of R₂ and S₂ is hydrogen.

In another specific embodiment, the invention relates to a sub-class of such monocyclic, bicyclic and polycyclic compounds having the general formula **1**, as well as a sub-class of such monocyclic compounds having the general formula **2**, wherein one of A and B is hydrogen and wherein the other of A and B is selected from the group consisting of hydrogen, halogen, azido, C=NOR₆, C=N-NR₇R₈, COOR₆, CONR₇R₈, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, arylalkyl, alkoxyaryl and heterocyclic, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, C₁₋₂₀ alkylamino, C₃₋₁₀ cycloalkylamino, C₂₋₂₀ alkenylamino, C₃₋₁₀ cycloalkenylamino, arylamino, arylalkylamino, heterocyclic amino, hydroxyalkylamino, mercaptoalkylamino and C₂₋₂₀ alkynylamino, acylamino and thioacylamino.

In another specific embodiment, the invention relates to a sub-class of such monocyclic and bicyclic compounds wherein each of S₁, S₂, S₃, S₄ and S₅ is hydrogen (i.e. each ring atom of the moiety represented by the general formula 2 is monosubstituted) and further wherein each of Q₁, Q₂, Q₃, Q₄ and Q₅ is hydrogen (i.e. each ring atom of the moiety represented by the general formula 3 is monosubstituted).

In another specific embodiment, the invention relates to a sub-class of such monocyclic, bicyclic and polycyclic compounds having the general formula 1, as well as a sub-class of such monocyclic compounds having the general formula 2, wherein no more than two of S₁, S₂, S₃, S₄ and S₅ is hydrogen and/or no more than two of Q₁, Q₂, Q₃, Q₄ and Q₅ is hydrogen.

All of the sub-possibilities listed above may be combined at will, i.e. each of the substituents A, B, R₁, R₂, R₃, R₄, R₅, S₁, S₂, S₃, S₄, S₅, Q₁, Q₂, Q₃, Q₄, Q₅, P₁, P₂, P₃, P₄ or P₅ may independently correspond to any of the definitions given above, in particular with any of the individual meanings (such as illustrated above) of generic terms used for substituting radicals such as, but not limited to, "C₁₋₂₀ alkyl ", " C₃₋₁₀ cycloalkyl ", " C₂₋₂₀ alkenyl ", " C₂₋₂₀ alkynyl", " aryl ", " homocyclic ", " heterocyclic ", " halogen ", " C₃₋₁₀ cycloalkenyl ", "alkylaryl ", "arylalkyl ", "alkylamino", " cycloalkylamino ", "alkenylamino ", "alkynylamino", "arylamino", " arylalkylamino ", "heterocyclic amino ", "hydroxyalkylamino ", " mercaptoalkylamino ", " alkynylamino ", " C₁₋₇ alkoxy", " C₃₋₁₀ cycloalkoxy ", "thio C₁₋₇ alkyl ", " thio C₃₋₁₀ cycloalkyl ", "halo C₁₋₇ alkyl ", "amino-acid " and the like. Similarly, the linker L may, in any combination with the above-listed substituents, correspond to any of the generic definitions given above, in particular with any of the individual meanings for such generic definitions.

Each of the compounds of the invention, whatever the general formula herein-before, may be in the E or Z stereoisomeric form.

Pure stereoisomeric forms of the bicyclic compounds of this invention may be obtained by using procedures known in the art. For instance, enantiomers may be separated from each other by the selective crystallization of their diastereomeric salts with optically active acids. Alternatively, enantiomers may be separated by chromatographic techniques known in the art, using chiral commercially available stationary phases. Diastereomers may be obtained by suitable conventional methods of physical separation such as, for example, selective crystallization or column chromatography.

The present invention also relates to methods for making the above-referred compounds.

These compounds may be prepared according to several methods, either via derivatization of simmondsin and its naturally occuring derivatives, via hemisynthesis from simmondsin and its naturally occuring derivatives, or via a fully synthetic route, as follows.

### Method 1 - synthesis via enzymatic derivatization of simmondsin

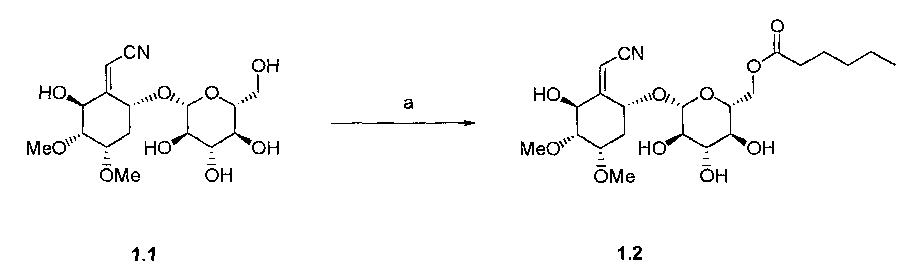

### (a) vinyl hexanoate and enzyme

Starting from natural simmondsin, it is possible to regioselectively introduce one or more acyl groups using enzymes in combination with an acyl donor and in the presence of one or more organic solvents. Examples of such enzymes are lipases, esterases, acylases and proteases. Examples of acyl donors are aromatic, heteroaromatic, aliphatic, alicyclic, heterocyclic carboxylic acids and their activated esters, e.g. vinyl esters, 2-propenyl esters, alkyl esters, chlorinated, brominated or fluorinated alkyl esters, or any other ester which can be used for this purpose as known to anybody skilled in the art. (see e.g. K. Faber, *"Biotransformations in organic synthesis"*, 3^{rd} edition, Springer Verlag, Berlin, 1997; Bornscheuer et al. in *"Hydrolases is organic synthesis. regio- and stereoselective biotransformations",* Wiley-VCH, New-York, 1999). The acyl donor may also be used as the solvent. Other typical solvents include, but are not limited to chloroform, toluene, ether solvents like tetrahydrofuran, diethyl ether, diisopropyl ether and ter-butyl methyl ether, or any other suitable solvent known to anybody skilled in the art.

### Method 2 - synthesis via chemical modification of simmondsin

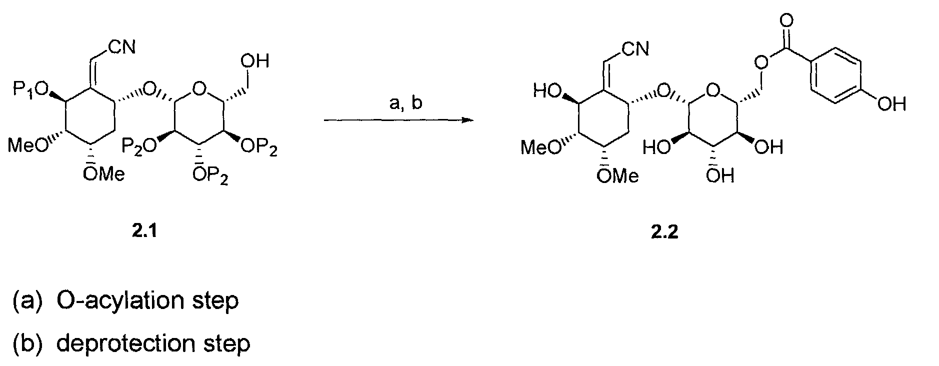

Natural or synthetic simmondsin **2.1** or one of its analogues is suitably protected by hydroxyl-protecting groups P₁ and P₂ such that only one hydroxyl group remains free. Examples of such protective groups P₁ and P₂ and the way to introduce them are well known in carbohydrate chemistry, and include, but are not limited to, the following classes: esters, acetals, ethers, silyl ethers, carbamates and carbonates (see e.g. T.K. Lindhorst, *"Essentials of carbohydrate chemistry and biochemistry",* Wiley-VCH, New-York, 2000; R.V. Stick, *"Carbohydrates. The sweet molecules of life",* Academia Press, New-York, 2001; G.J. Boons, *"Carbohydrate chemistry",* Blackie Academic and Professional, Thomas Science London, 1998; T.W. Greene, P.G.M. Wuts, *"Protective groups in organic synthesis"*, 3^{rd} edition, J. Wiley & Sons, New-York, 1999). Subsequently, the free hydroxyl group is e.g. acylated or alkylated in order to introduce the desired side chain. O-acylation can be performed using e.g. carboxylic acid chlorides, carboxylic acid anhydrides or activated carboxylic acid esters including, but not limited to, paranitrophenyl esters and succinimidyl esters.

Alternatively, a carboxylic acid can be transformed into an active ester *in situ* using an activating agent such as **N**,**N**'-dicyclohexylcarbodiimide (DCC) in the presence of N-hydroxy-benzotriazole (HOBt) or any other activating reagent known to anybody skilled in the art.

O-alkylation is typically performed by deprotonating the free hydroxyl group, e.g. with sodium hydride (NaH) or potassium t.butoxide (KOt.Bu), in a dry aprotic solvent (typically an ether solvent like diethyl ether, tetrahydrofuran or dimethoxyethane), and subsequently treating the alcoholate with the desired alkyl halide or alkyl sulfonate. A dipolar aprotic solvent, e.g. dimethyl sulfoxide (DMSO), hexamethyl phosporic triamide (HMPA) or 1,3-dimethyl-3,4,5,6-tetrahydro-2(1*H*)-pyrimidinone (DMPU), or a crown ether, e.g. 15-crown-5 or 18-crown-6, can be added to accelerate the reaction.

Finally, careful deprotection in step (b), using methods standard in the art, affords the desired compound represented by the formula **2.2.**

### Method 3 - synthesis of stereoisomers via chemical modification

Starting from a suitably protected simmondsin analogue (such as described with respect to method 2 above), the configuration at the carbon atom carrying the free hydroxyl group can be inverted by one of the following methods: 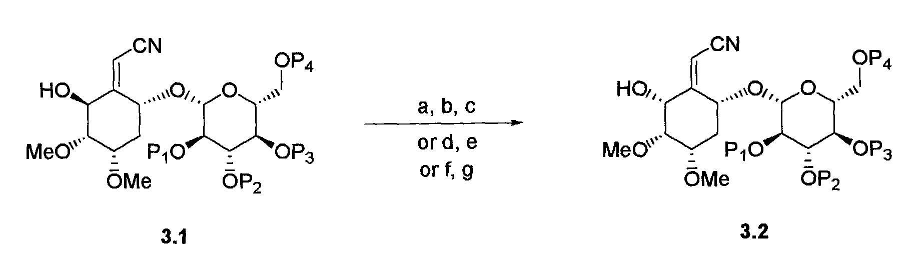
- by conversion of the alcohol function into a good leaving group, preferably a sulfonate, e.g. a mesylate, triflate, tosylate, nosylate or brosylate, in step (a), followed by substitution with a nucleophile in step (b). Examples of such nucleophiles include, but are not limited to, a carboxylate anion, an amine, a hydrazine, a hydroxylamine, a sulfide, a fluoride. In the above illustrative but non limiting scheme, acetate is used as the nucleophile in step (b), followed by ester hydrolysis in step (c). Such a substitution reaction is best carried out in a dipolar aprotic solvent, e.g. N,N-dimethylformamide (DMF), dimethyl sulfoxide (DMSO), hexamethyl phosporic triamide (HMPA), N-methyl-pyrrolidone (NMP), 1,3-dimethyl-3,4,5,6-tetrahydro-2(1*H*)-pyrimidinone (DMPU), or in an ether solvent (e.g. diethyl ether, tetrahydrofuran, 1,2-dimethoxyethane, 1,4-dioxane) with a dipolar aprotic solvent as an optional co-solvent. The required sulfonates may be obtained by treating the free alcohol function with the corresponding sulfonyl chloride in a halogenated solvent (e.g. methylene chloride, ethylene chloride) in the presence of pyridine or triethylamine as a base, and a nucleophilic catalyst, e.g. N,N-dimethylaminopyridine or N-methyl-imidazole. Alternatively, the sulfonylation reaction can also be performed in pyridine as solvent.
- by an oxidation-reduction sequence: oxidation of the free alcohol function in step (d), followed by reduction in step (e) can lead to inversion of the configuration. Typical oxidative agents include, but are not limited to pyridinium chlorochromate (PCC), pyridinium dichromate (PDC), tetrapropylammonium perruthenate (TPAP) / N-methylmorpholine-N-oxide, periodinane, dimethylsulfoxide (DMSO) / oxalyl chloride / triethylamine (Swern oxidation). An example of a typical solvent for such oxidation is methylene chloride. Typical reductive agents include, but are not restricted to, sodium borohydride (in methanol), zinc borohydride (in ether or DME), lithium aluminum hydride (in ether or THF), lithium tris(ter-butoxy)-aluminum hydride (in ether or THF), diisobutyl aluminum hydride (DIBAH; in ether, THF, DME, toluene or methylene chloride).
- by a Mitsunobu inversion (e.g. following the teaching of O. Mitsunobu in *Synthesis*, **1981,** 1-28): typically, activation of the alcohol function with diethyl azodicarboxylate (DEAD) and triphenyl phosphine in anhydrous THF, in the presence of a carboxylic acid as a nucleophile affords the corresponding ester via nucleophilic substitution with inversion of configuration in step (f), followed by hydrolysis leading to the inverted alcohol in step (g). In a similar way, a phenol can also be used, giving rise to the corresponding phenyl ether with inversion of configuration. Other nucleophiles, e.g. iodide, azide or sulfides may also be introduced using the Mitsunobu activation method, by replacing the carboxylic acid with zinc iodide, zinc azide or 2-mercaptobenzothiazole as a nucleophile (see e.g. T.K. Lindhorst, *"Essentials of carbohydrate chemistry and biochemistry",* Wiley-VCH, New-York, 2000).

### Method 4 - preparation of simmondsin aglucone

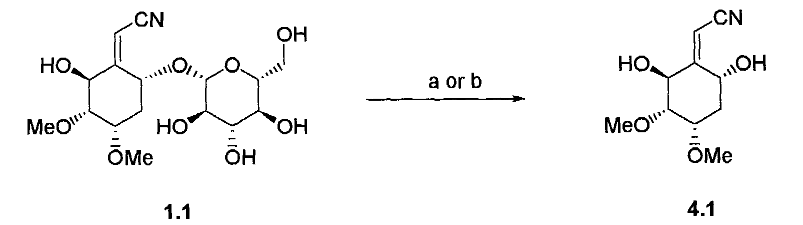

Treatment of simmondsin **1.1** or its desmethyl analogue under mild conditions with dilute mineral acid in water or an alcohol causes cleavage of the anomeric linkage in step (a). Alternatively, cleavage may be catalyzed by enzymes such as glycosidases, more in particular in this case β-D-glucosidases in step (b).

Starting from a suitably protected simmondsin (see e.g. Greene et al. in *"Protective groups in organic synthesis"*, 3^{rd} edition, J. Wiley & Sons, New-York, 1999), the aglucone will have only one free hydroxyl function.

### Method 5 - preparation of a simmondsin analogue from an aglucone

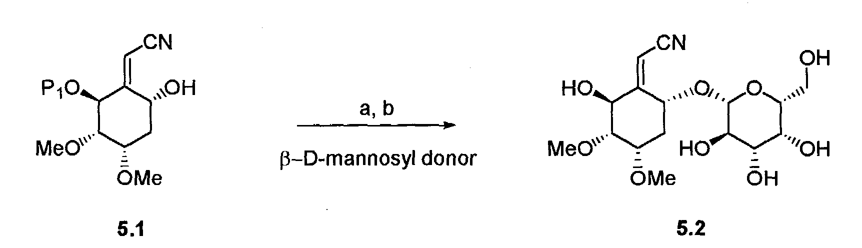

A suitably protected simmondsin aglucone **5.1** may be coupled with any suitable glycosyl donor in step (a), using coupling methods well known in the art, followed by deprotection in step (b). The leaving group of the glycosyl donor may be acetate (L = OAc), bromide (L = Br), chloride (L = CI), fluoride (R = F), trichloroacetimidate (L = OC(NH)CCl₃), sulfide (L = SR), 4-pentenyl, or any other activating group known to anybody skilled in the art (see e.g. T.K. Lindhorst, *"Essentials of carbohydrate chemistry and biochemistry",* Wiley-VCH, New-York, 2000). This reaction may be activated using known Lewis acids like BF₃.Et₂O, SnCl₄, TMSOTf.

### Method 6

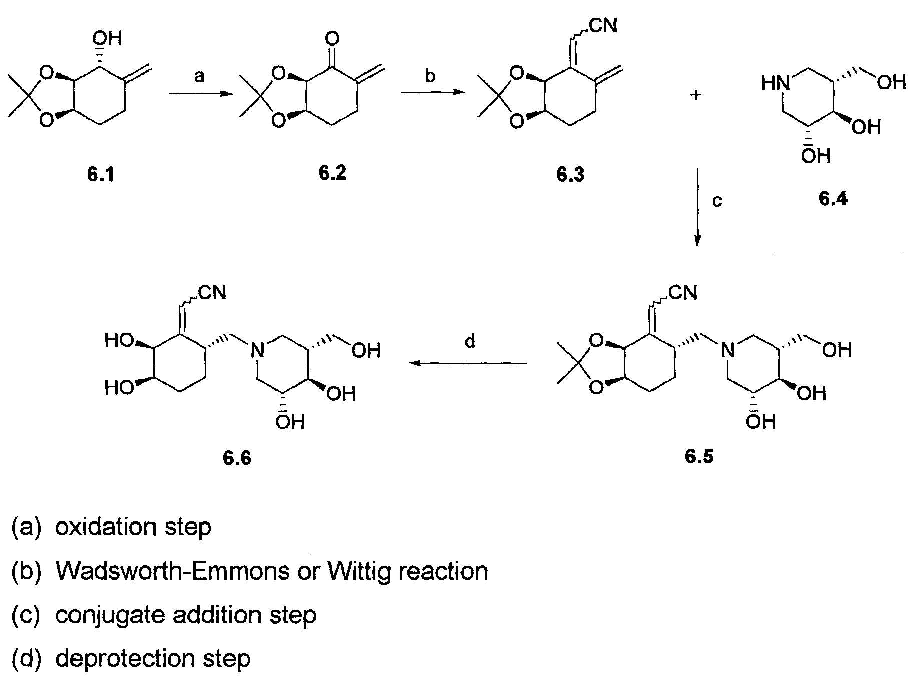

This methods proceeds in a sequence of five steps as illustrated by the above scheme. The synthesis of the starting material **6.1** for step (a) is known in the art, e.g. from Hubrecht et al. in *Synlett,* **2000**, *7*, 971-974. The synthesis of the starting material **6.4** (isofagomine) for step (c) is known in the art, e.g. from J. Andersch et al. in *Chem. Eur. J.,* **2001,** *7* (17), 3744-3747.

### Method 7

This methods proceeds in a sequence of steps as illustrated by this scheme.

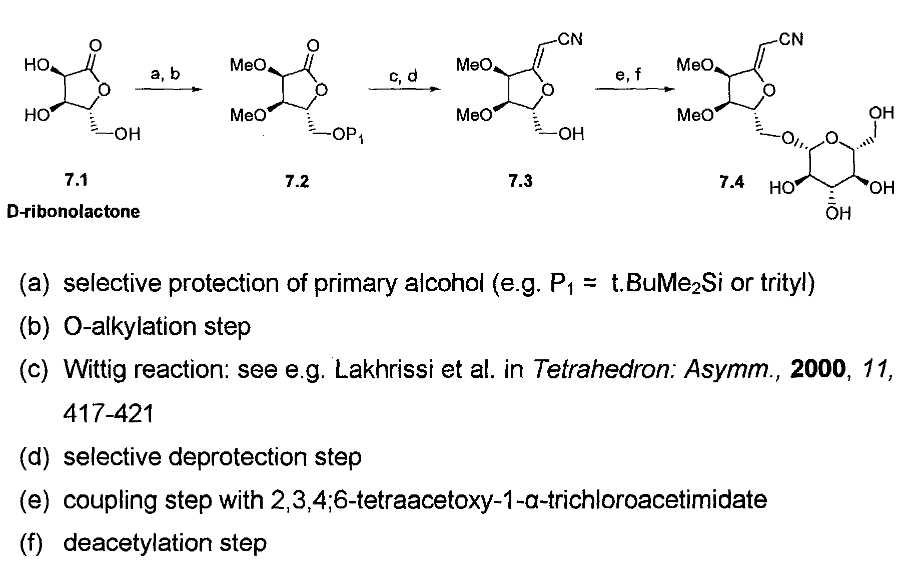

In another aspect, the present invention also provides a pharmaceutical composition comprising one or more pharmaceutically acceptable excipients and an effective amount of a bicyclic compound having the general formula **1**, a salt, a solvate, a stereoisomer or an enantiomer thereof. Such a pharmaceutical composition may be useful, among others, for inhibiting angiogenesis or for treating angiogenesis-related diseases in mammals, preferably in humans, or other animals, or for inhibiting feeding in animals.

The present invention further relates to a method of inhibiting angiogenesis or treating angiogenesis-related diseases in mammals, preferably in humans, and other animals, said method comprising administering to the mammal, preferably human, or other animal in need thereof an angiogenesis-inhibiting effective amount of a bicyclic compound having the general formula **1**, a salt, a solvate, a stereoisomer or an enantiomer thereof.

The present invention also relates to a method of inhibiting feeding in mammals, preferably in humans, and other animals, said method comprising administering to said mammal, preferably human, or other animal a feeding-inhibiting effective amount of a bicyclic compound having the general formula **1**, a salt, a solvate, a stereoisomer or an enantiomer thereof.

It has now surprisingly been found that bicyclic compounds such as defined above, and in particular compounds having the general formula **1** have a potent angiogenesis-inhibiting effect.

Endothelial cells, that form the walls of blood vessels, are the source of new blood vessels and have a great ability to divide and travel. The construction of a vascular network requires different sequential steps including:
1. the release of proteases from activated endothelial cells,
2. a degradation of the basement membrane surrounding the existing vessel,
3. a migration of the endothelial cells into the interstitial space,
4. endothelial cell proliferation,
5. tube formation and the subsequent lumen formation,
6. generation of new basement membrane with the recruitment of pericytes,
7. fusion of the newly formed vessels, and
8. initiation of blood flow.

Surprisingly, bicyclic compounds according to this invention show a significant activity on one or more of the above sequential steps involved in the angiogenesis pathway.

Angiogenesis plays an important role in a number of diseases. For instance, angiogenesis is important in the development of many malignancies in colon, breasts, endometrium, ovary, cervix, prostate and other tissues. Since the present invention provides compounds useful for inhibiting angiogenesis, these compounds are also particularly suitable for treating diseases that result from unwanted or uncontrolled angiogenesis activity.

Angiogenesis is of particular interest in cancer. *De novo* capillary production is a crucial event in tumor growth and metastasis since the cells in solid tumors must receive the necessary oxygen and nutrients to survive and grow. In particular, it is known that tumors depending on angiogenesis represent a large number of the existing cancers. Inhibition of blood vessel development results in restricted energy supply to the tumor, thus causing an arrest in its development and hence, the start of its regression. In view of the effects of angiogenesis in cancer development, the present invention provides compounds having anti-angiogenesis activity which are useful in the treatment of cancers depending on angiogenesis.

Unexpectedly, bicyclic compounds according to this invention also have anti-tumor activity. In addition, bicyclic compounds according to this invention surprisingly show low levels of cytotoxity *in vitro*, meaning that they do not induce significant detrimental effect(s) on healthy cells, tissues or organs. Also, bicyclic compounds according to this invention surprisingly show no significant oestrogen-like activity, meaning that their administration does not imply a risk of boosting cancer tumors in women.

Therefore bicyclic compounds according to this invention may be safely used for the manufacture of a medicament for treating cancer. Cancers which may be treated according to the present invention include, but are not limited to, solid tumors or metastasis, i.e. the form of cancer wherein the transformed or malignant cells are traveling and spreading cancer from one organ to another. The relevant cancer may relate to one of the following organs: heart, lung, intestine, genito-urinary tract, liver, bone, nervous system, blood, skin, breast, brain, and adrenal glands. More particularly, the bicyclic compounds according to this invention may be used for treating gliomas (e.g. Schwannoma, glioblastoma, astrocytoma), melanoma, neuroblastoma, pheochromocytoma, paraganlioma, meningioma, adrenalcortical carcinoma, kidney cancer, vascular cancer, osteoblastic osteocarcinoma, prostate cancer, ovarian cancer, uterine leiomyomas, salivary gland cancer, choroid plexus carcinoma, mammary cancer, pancreatic cancer, colon cancer, and megakaryoblastic leukemia.

Recent studies also suggest angiogenesis-inhibiting compounds to be effective in the treatment of other diseases where uncontrolled blood vessel growth and synovial inflammation is involved, such as arthritis. Therefore, the bicyclic compounds of the present invention may be efficiently used for the manufacture of a medicament for treating arthritis as well as for the manufacture of a medicament for treating psoriaris. The term " psoriasis " is defined herein as being a chronic skin disease characterized by scaling and inflammation and including plaque psoriasis, guttate psoriasis, pustular psoriasis, inverse psoriasis, erythrodermic psoriasis and the like.

Since angiogenesis-inhibition plays a role in placenta and embryonic development, the present active compoounds can also be used, according to another embodiment of the invention, for contraceptive and abortive purposes.

The pharmaceutical compositions of this invention usually contain from about 0.1 % to 50 % by weight, preferably from 1 % to 40 % by weight, more preferably from 5 % to 30 % by weight, of the active bicyclic compounds. The pharmaceutical preparations can be prepared in a manner known per se to a person skilled in the art. For this purpose, at least one active component according to the invention is admixed with one or more pharmaceutical excipients and/or auxiliaries and, if desired, in combination with other pharmaceutically active components.

Suitable pharmaceutical carriers for use in the said pharmaceutical compositions and their formulation are well known to those skilled in the art. There is no particular restriction to their selection within the present invention although special attention should be paid to the selection of suitable carrier combinations that can assist in properly formulating them in view of the expected time release profile. Suitable pharmaceutical carriers include additives such as wetting agents, dispersing agents, stickers, adhesives, emulsifying or surface-active agents, thickening agents, complexing agents, gelling agents, solvents, coatings, antibacterial and antifungal agents (for example phenol, sorbic acid, chlorobutanol), isotonic agents (such as sugars or sodium chloride) and the like, provided the same are consistent with pharmaceutical practice, i.e. carriers and additives which do not create permanent damage to mammals. The pharmaceutical compositions of the present invention may be prepared in any known manner, for instance by homogeneously mixing, dissolving, spray-drying, coating and/or grinding the active ingredients, in a one-step or a multi-steps procedure, with the selected carrier material and, where appropriate, the other additives such as surface-active agents. may also be prepared by micronisation, for instance in view to obtain them in the form of microspheres usually having a diameter of about 1 to 10 µm, namely for the manufacture of microcapsules for controlled or sustained release of the biologically active ingredient(s).

Suitable surface-active agents to be used in the pharmaceutical compositions of the present invention are non-ionic, cationic and/or anionic materials having good emulsifying, dispersing and/or wetting properties. Suitable anionic surfactants include both water-soluble soaps and water-soluble synthetic surface-active agents. Suitable soaps are alkaline or alkaline-earth metal salts, unsubstituted or substituted ammonium salts of higher fatty acids (C₁₀-C₂₂), e.g. the sodium or potassium salts of oleic or stearic acid, or of natural fatty acid mixtures obtainable form coconut oil or tallow oil. Synthetic surfactants include sodium or calcium salts of polyacrylic acids; fatty sulphonates and sulphates; sulphonated benzimidazole derivatives and alkylarylsulphonates. Fatty sulphonates or sulphates are usually in the form of alkaline or alkaline-earth metal salts, unsubstituted ammonium salts or ammonium salts substituted with an alkyl or acyl radical having from 8 to 22 carbon atoms, e.g. the sodium or calcium salt of lignosulphonic acid or dodecylsulphonic acid or a mixture of fatty alcohol sulphates obtained from natural fatty acids, alkaline or alkaline-earth metal salts of sulphuric or sulphonic acid esters (such as sodium lauryl sulphate) and sulphonic acids of fatty alcohol/ethylene oxide adducts. Suitable sulphonated benzimidazole derivatives preferably contain 8 to 22 carbon atoms. Examples of alkylarylsulphonates are the sodium, calcium or alcanolamine salts of dodecylbenzene sulphonic acid or dibutyl-naphtalenesulphonic acid or a naphtalene-sulphonic acid/formaldehyde condensation product. Also suitable are the corresponding phosphates, e.g. salts of phosphoric acid ester and an adduct of p-nonylphenol with ethylene and/or propylene oxide, or phospholipids. Suitable phospholipids for this purpose are the natural (originating from animal or plant cells) or synthetic phospholipids of the cephalin or lecithin type such as e.g. phosphatidylethanolamine, phosphatidylserine, phosphatidylglycerine, lysolecithin, cardiolipin, dioctanyl-phosphatidylcholine, dipalmitoylphoshatidylcholine and their mixtures.

Suitable non-ionic surfactants include polyethoxylated and polypropoxylated derivatives of alkylphenols, fatty alcohols, fatty acids, aliphatic amines or amides containing at least 12 carbon atoms in the molecule, alkylarenesulphonates and dialkylsulphosuccinates, such as polyglycol ether derivatives of aliphatic and cycloaliphatic alcohols, saturated and unsaturated fatty acids and alkylphenols, said derivatives preferably containing 3 to 10 glycol ether groups and 8 to 20 carbon atoms in the (aliphatic) hydrocarbon moiety and 6 to 18 carbon atoms in the alkyl moiety of the alkylphenol. Further suitable non-ionic surfactants are water-soluble adducts of polyethylene oxide with poylypropylene glycol, ethylenediaminopolypropylene glycol containing 1 to 10 carbon atoms in the alkyl chain, which adducts contain 20 to 250 ethyleneglycol ether groups and/or 10 to 100 propyleneglycol ether groups. Such compounds usually contain from 1 to 5 ethyleneglycol units per propyleneglycol unit. Representative examples of non-ionic surfactants are nonylphenol-polyethoxyethanol, castor oil polyglycolic ethers, polypropylene/ polyethylene oxide adducts, tributylphenoxypolyethoxyethanol, polyethyleneglycol and octylphenoxypolyethoxyethanol. Fatty acid esters of polyethylene sorbitan (such as polyoxyethylene sorbitan trioleate), glycerol, sorbitan, sucrose and pentaerythritol are also suitable non-ionic surfactants.

Suitable cationic surfactants include quaternary ammonium salts, preferably halides, having 4 hydrocarbon radicals optionally substituted with halo, phenyl, substituted phenyl or hydroxy; for instance quaternary ammonium salts containing as N-substituent at least one C₈-C₂₂ alkyl radical (e.g. cetyl, lauryl, palmityl, myristyl, oleyl and the like) and, as further substituents, unsubstituted or halogenated lower alkyl, benzyl and/or hydroxy-lower alkyl radicals.

A more detailed description of surface-active agents suitable for this purpose may be found for instance in "McCutcheon's Detergents and Emulsifiers Annual" (MC Publishing Crop., Ridgewood, New Jersey, 1981), "Tensid-Taschenbuch", 2^{nd} ed. (Hanser Verlag, Vienna, 1981) and "Encyclopaedia of Surfactants (Chemical Publishing Co., New York, 1981).

Structure-forming, thickening or gel-forming agents may be included into the pharmaceutical compositions and combined preparations of the invention. Suitable such agents are in particular highly dispersed silicic acid, such as the product commercially available under the trade name Aerosil; bentonites; tetraalkyl ammonium salts of montmorillonites (e.g., products commercially available under the trade name Bentone), wherein each of the alkyl groups may contain from 1 to 20 carbon atoms; cetostearyl alcohol and modified castor oil products (e.g. the product commercially available under the trade name Antisettle).

Gelling agents which may be included into the pharmaceutical compositions and combined preparations of the present invention include, but are not limited to, cellulose derivatives such as carboxymethylcellulose, cellulose acetate and the like; natural gums such as arabic gum, xanthum gum, tragacanth gum, guar gum and the like; gelatin; silicon dioxide; synthetic polymers such as carbomers, and mixtures thereof. Gelatin and modified celluloses represent a preferred class of gelling agents.

Other optional excipients which may be included in the pharmaceutical compositions and combined preparations of the present invention include additives such as magnesium oxide; azo dyes; organic and inorganic pigments such as titanium dioxide; UV-absorbers; stabilisers; odor masking agents; viscosity enhancers; antioxidants such as, for example, ascorbyl palmitate, sodium bisulfite, sodium metabisulfite and the like, and mixtures thereof; preservatives such as, for example, potassium sorbate, sodium benzoate, sorbic acid, propyl gallate, benzylalcohol, methyl paraben, propyl paraben and the like; sequestering agents such as ethylene-diamine tetraacetic acid; flavoring agents such as natural vanillin; buffers such as citric acid and acetic acid; extenders or bulking agents such as silicates, diatomaceous earth, magnesium oxide or aluminum oxide; densification agents such as magnesium salts; and mixtures thereof.

Additional ingredients may be included in order to control the duration of action of the biologically-active ingredient in the compositions and combined preparations of the invention. Control release compositions may thus be achieved by selecting appropriate polymer carriers such as for example polyesters, polyamino-acids, polyvinyl-pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethyl-cellulose, polymethyl methacrylate and the other above-described polymers. Such methods include colloid drug delivery systems like liposomes, microspheres, microemulsions, nanoparticles, nanocapsules and so on. Depending on the route of administration, the pharmaceutical composition or combined preparation of the invention may also require protective coatings.

Pharmaceutical forms suitable for injectable use include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol, complexing agents such as cyclodextrins and the like, and mixtures thereof.

In a preferred embodiment the administration route of a pharmaceutical composition according to the present invention is an oral, a topical or a parenteral route.

According to one embodiment the pharmaceutical composition according to the invention is formulated to be applied orally to humans and animals. The present pharmaceutical composition can for instance by applied orally for the treatment of tumors or other angiogenesis-related diseases.

In yet another embodiment, the pharmaceutical composition according to the invention is formulated to be applied topically. The present pharmaceutical composition can for instance by applied topically for the treatment of tumors or other angiogenesis-related diseases, e.g. on the skin of humans and animals. Preferably, said pharmaceutical composition for topical administration includes the bicyclic compound of this invention preferably in a concentration ranging from about 0.1 to 10 % by weight, preferably from 0.5 to 5 % by weight.

Active bicyclic compounds according to the invention can also be lyophilized, for example for the production of injection or infusion preparations. Suitable solvents for this purpose include water, physiological saline solution or alcohols (e. g. ethanol, isopropanol or glycerol), saccharide solutions such as glucose or mannitol solutions, or mixtures thereof. Injectable solutions or suspensions may be formulated by using suitable non-toxic, parenterally-acceptable diluents or solvents, such as mannitol, 1,3-butanediol, water, isotonic sodium chloride solution, and/or suitable dispersing or wetting and suspending agents, such as synthetic mono- or diglycerides and fatty acids (e.g. oleic acid).

The dose of an active component according to the invention, to be administered depends on the individual case and upon the frequency of administration, but also on the nature and severity of the disease and symptoms, as well as the age, weight and individual responsiveness of the patient.

The present invention will now be described with reference to examples that are by no means limiting the protection of the invention and are given only with the purpose of enabling one skilled in the art.

The following examples provide methods for making the monocyclic and bicyclic compounds of the invention, as well as methods and assays for evidencing their angiogenesis-inhibiting activity.

### Example 1 - chorion allantois membrane (CAM) assay

The following CAM assay is based on the teaching of Nguyen et al. in *Microvasc. Res.* (1994) 47 (1):31-40. Fertilized chicken eggs are incubated during 4 days at 37 °C. Subsequently, the egg shell is opened to disclose the CAM. In order to reduce the pressure on the CAM for improving its manipulating properties, 2 to 3 ml albumen is removed. The opening is closed using cellophane tape and the eggs are further incubated until day 9 for application of the test compounds using different concentrations.

Dulbecco's modified Eagle medium (DMEM) is used as a negative control, whereas Vascular Endothelial Growth Factor (hereinafter referred as VEGF) is used as a positive control (i.e. a stimulator of angiogenesis). A compound of the invention and tangeritin, a commercially known angiogenesis inhibitor, are compared in their angiogenesis-inhibiting activity towards VEGF stimulated angiogenesis in this assay. Each of the solubilized test compositions is pipetted on a sterile plastic disc (10 mm diameter) and dried under sterile conditions.

Each disc is treated with cortisone acetate (2 µg/µl in a suitable buffer) in order to avoid inflammatory reactions. After drying, the discs are placed on the CAM membrane with the drug loaded side facing the membrane. The control disc (DMEM) is placed at 10 mm distance from the disc containing the test compound. The opening is sealed with cellophane tape and the eggs are further incubated. At day 11, the tape and discs are removed and 10% buffered formaline is sprayed over the membrane. Eggs are allowed to dry for 4 hours at room temperature. Then, the CAM membrane is cut out of the egg and mounted on a glass slide. The vascular density index is then measured according to the method disclosed by Harris-Hooker et al. in J. *Cell. Physio.* (1983) 114: 302-310), while counting all micro vessels that cross 3 concentric circles with a diameter of 6 ,8 and 10 mm. An angiogenetic index (Al) is expressed as { (t/c) x100 } -100, wherein t is the total number of crossing vessels where the disc containing the test compound is located and c is the total number of crossing vessels where the control disc is located. The test compound may be classified positively when Al exceeds 10 and negatively when Al is below 10.

### Example 2 - assays based on human vascular endothelial cells (HUVEC)

The following illustrates a series of assays based on HUVEC for determining the angiogenesis-inhibiting effects of the compounds of the invention.

Human Vascular Endothelial Cells (HUVEC) are obtained as described by Jaffe et al. in *J. Clin. Invest*. (1973) 52:2745-2746 and cultured to confluence on fibronectin-coated dishes in M199 medium supplemented with 20 mM HEPES (pH 7.3), 10 % human serum, 10 % heat-inactivated new born calf serum (NBCS),150 µg/ml crude endothelial cell growth factor (ECGF), 2 mM L-glutamine, 5 U/ml heparin, 100 U/ml penicillin and 100 µg/ml streptomycin at 37 °C under a 5% CO₂ / 95% air atmosphere. The confluent HUVEC cultures (passage 0) are detached by means of trypsin / EDTA treatment, pooled and cultured after a split ratio of 1:3 till confluence (passage 1). Then the pooled HUVEC are frozen in 10 cm³ aliquots in vials in liquid nitrogen. One week before proliferation, vials of HUVEC (passage 1) are thawed and cultured (after a split ratio of 1: 3) to confluence (passage 2).

### Experiment A: cell proliferation measured by 3H-thymidine incorporation

Confluent cultures of HUVEC (passage 2) are detached by means of a trypsin / EDTA solution, and allowed to adhere and spread at an appropriate cell density on gelatin-coated dishes in a M199-HEPES medium supplemented with 10 % heat-inactivated NBCS, 100 U/ml penicillin and 100 µg/ml streptomycin. After 18 hours, HUVEC are stimulated with 6.25 ng/ml vascular endothelial growth factor type A (VEGF-A) in M199-HEPES, 100 IU/ml penicillin, 100 pg/ml streptomycin and 10 % NBCS in duplicate wells, with or without the test compound of the invention. After an incubation period of 48 hours, a tracer amount (0.5 µCi/well) of [3H]-thymidine is added and cells are incubated for another 6 hours. Subsequently, the cells are washed with PBS, [3H]-labelled DNA is fixed with methanol, precipitated in 5 % trichloroacetic acid, and finally dissolved in 0.5 ml 0.3 M NaOH and counted in a liquid scintillation counter. The level of [3H]-thymidine incorporation provides a quantitative indication of the inhibition of VEGF-a-induced HUVEC proliferation by the compounds of the invention.

### Experiment B: cell proliferation measured by cell counting

A confluent monolayer of HUVEC is detached by means of a trypsin /EDTA solution, and allowed to adhere and spread at cell density of 15% confluency in gelatine-coated flasks in M199-HEPES medium supplemented with 10% heat-inactivated NBCS and 100 IU/ml penicillin and 100 pg/ml streptomycin. After 18 hours the HUVEC are stimulated with 2.5 ng/ml basic fibroblast growth factor (FGF) in M199 medium-HEPES supplemented with penicillin/streptomycin, 10 % NBCS and 0.1% DMSO in triplicate wells for 6 days. The cell number is determined by image analysis. This experiment provides evidence for the anti-angiogenic effect of the compounds of the invention.

### Experiment C: in vitro angiogenesis assay, tube formation in 3-D fibrin matrices

Human fibrin matrices are prepared by adding 0.1 U/ml thrombin to a commercial (Chromogenix AB, Sweden) mixture of 2 mg/ml fibrinogen (final concentrations), 2 mg/ml Na-citrate, 0.8 mg/ml NaCl, 3 µg/ml plasminogen in M199 medium and 2.5 U/ml factorXIII. 100 pl aliquots of this mixture are added to the wells of 96-well plates. After clotting at room temperature, the fibrin matrices are soaked with M199 supplemented with 10 % human serum and 10 % NBCS for 2 hours at 37 °C in order to inactivate thrombin. Frozen human microvascular endothelial cells (hMVEC, 0.7 x10⁵ cells/cm²) are thawed and seeded in a 1.8:1 split ratio on the fibrin matrices and cultured for 24 hours in M199 medium supplemented with 10 % human serum, 10% NBCS, and 100 IU/ml penicillin and 100 µg/ml streptomycin. Then, hMVEC are stimulated with a compound of the invention for 7 days. Fresh medium, containing said compound, are added every second day. Invading cells and the formation of tubular structures of hMVEC in the three-dimensional fibrin matrix are then analyzed by phase contrast microscopy. The total length of tube-like structures of four microscopic fields (7.3 mm²/field) are measured using an Olympus CK2 microscope equipped with a monochrome CCD camera (MX5) connected to a computer with Optimas image analysis software, and expressed as mm/cm² according to the teaching of Koolwijk et al. in J. *Cell Biol.* (1996) 132:1177-1188.

### Experiment D - ex vivo angiogenesis assay

Such an assay may be performed as described by Deckers et al. in *Lab*. *Invest.* (2001) 81:5-15. 17 days-old fetus are removed from pregnant Swiss albino mice and metatarsals are dissected. The isolated metatarsals are cultured in 24-well plates in 150 µl minimal essential medium (aMEM) supplemented with 10 % by volume heat-inactivated fetal bovine serum, 100 IU/ml penicillin and 100 µg/ml streptomycin for 72 hours. After this adhesion phase, metatarsals are attached to the culture plastic and medium is replaced by 250 µl fresh medium + 10 ng/ml recombinant human VEGF-A in the presence or absence of a compound of the invention. Metatarsals are cultured for 14 days, the medium being replaced every 7 days. At the end of the culture period, cultures were fixed in ZnMF fixative for 15 minutes at room temperature and subsequently stained for PECAM-1. Analysis allows the determination of the effect of the tested compound on VEGF-induced *ex vivo* tube formation from fetal mouse metacarpals.

### Experiment E : In vivo matrigel chamber assay

This assay may be performed as described by Kragh et al. in *Int. J. Oncol*. (2003) 22:305-311. Plexiglas ring/nylon net filter-chambers (0.2 ml) containing growth factor-reduced Matrigel and 200 ng basic fibroblast growth factor (βFGF) are subcutaneously implanted into the flanks of 3-months old FVB/N mice. Mice are administered a compound of the invention. Chamber angiogenesis is scored superficially on day 14 post-implantation.

The above-listed experiments demonstrate that compounds of the invention are able to:
(i) inhibit VEGF-and βFGF- induced human endothelial cells proliferation,
(ii) inhibit VEGF-induced in vitro tube formation by human microvascular endothelial cells in 3-dimensional fibrin matrices,
(iii) inhibit the ex vivo outgrowth of tube-like structures of endothelial cells from fetal mouse metacarpals, and
(iv) inhibit in vivo neovascularization of matrigel chambers in mice.

### Example 3 - oestrogenic bioassay

Yeast cells *(Saccharomyces cerevisiae*) containing a human oestrogen receptor (100 µl) is added to 25 ml of a growth medium comprising:
- D-glucose (2.5 ml from a 0.25 g/ml aqueous solution of D-glucose);
- L-aspartic acid (0.625 ml from a 4 g/l aqueous solution);
- vitamin solution (0.250 ml from a mixture comprising 8 mg thiamine, 8 mg pyridoxine, 8 mg panthotenic acid, 40 mg inositol and 20 ml biotin solution (2 mg/100 ml in water) in 180 ml water);
- L-threonine (0.2 ml from a 2.4 g/100 ml aqueous solution);
- Copper (II) sulfate (0.0625 ml from a 3.2 g in/I aqueous solution); and
- minimal medium (22.5 ml from 1l water including 13.61 g potassium dihydrogenophosphate, 1.98 g ammonium sulfate, 4.2 g potassium hydroxide, 1 ml of a 0.8 g/l aqueous solution of iron (III) sulfate, 50 mg L-leucine, 50 mg L-histidine, 50 mg adenine, 20 mg L-arginine hydrochloride, 20 mg L-methio-nine, 30 mg L-tyrosine, 30 mg L-isoleucine, 30 mg L-lysine hydrochloride, 25 mg L-phenylalanine, 100 mg L-glutamic acid, 150 mg L-valine, 375 mg L-serine and 0.25 ml chlorophenol red beta-D- galactopyranoside).
17 beta-oestradiol (20 µl aliquots from 0.008 nM to 100 nM, prepared from 10 mg in 10ml ethanol) and a test compound of the invention, in concentrations of 1, 10, 100 and 1000 µM respectively, are pipetted into 96-well plates in a sterile flow hood. The solvent is left to evaporate for about 40 minutes. The growth medium containing yeast is then s dispensed to all wells, except blank wells, in aliquots of 100 µl. The plates are incubated at 32 °C in a humidified atmosphere for 3 days, and then read at 540 / 360 nm by a microtiter plate reader. This experiment makes it possible to determine the presence or absence of oestrogen-like activity in the compounds of the invention.

## Claims

1. A compound having the general formula 1 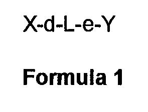 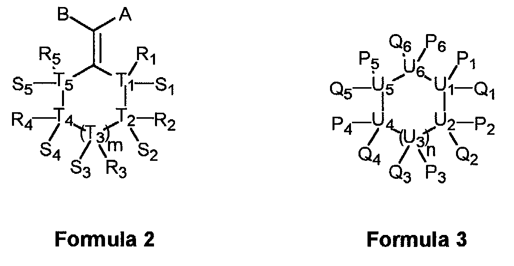 wherein:
- X is a moiety represented by the general formula 2 wherein:
- A and B are each independently selected from the group consisting of hydrogen, cyano, halogen, azido, C=NOR₆, C=N-NR₇R₈, COOR₆, CONR₇R₈, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, arylalkyl, alkoxyaryl and heterocyclic, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, C₁₋₂₀ alkylamino, C₃₋₁₀ cycloalkylamino, C₂₋₂₀ alkenylamino, C₃₋₁₀ cycloalkenylamino, arylamino, arylalkylamino, heterocyclic amino, hydroxyalkylamino, mercaptoalkylamino and C₂₋₂₀ alkynylamino, acylamino, thioacylamino, or A and B together form a homocyclic or heterocyclic group, provided that A and B are not both hydrogen,
- T₁, T₂, T₃, T₄ and T₅ are carbon atoms, or alternatively one of T₁, T₂, T₃, T₄ and T₅ is oxygen or nitrogen while the remaining of T₁, T₂, T₃, T₄ and T₅ are carbon atoms, or alternatively two of T₁, T₂, T₃, T₄ and T₅ are either both nitrogen, or oxygen and nitrogen, while the remaining of T₁, T₂, T₃, T₄ and T₅ are carbon atoms, or alternatively two of non adjacent T₁, T₂, T₃, T₄ and T₅ are oxygen while the remaining of T₁, T₂, T₃, T₄ and T₅ are carbon atoms,
- R₁, R₂, R₃, R₄ and R₅ are each substituents independently selected from the group consisting of hydrogen, cyano, halogen, azido, hydroxyl, amino, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, (di)C₁₋₂₀ alkylamino(thio)carbonyloxy, C₃₋₁₀ cycloalkylaminocarbonyloxy, C₁₋₂₀ alkylthio, C₃₋₁₀ cycloalkylthio, arylthio, arylalkylthio, acylthio, thio-heterocyclic, C₁₋₂₀ alkylamino, C₃₋₁₀ cycloalkylamino, C₂₋₂₀ alkenylamino, C₃₋₁₀ cycloalkenylamino, arylamino, arylalkylamino, heterocyclic amino, hydroxyalkylamino, mercaptoalkylamino, C₂₋₂₀ alkynylamino, acylamino, thioacylamino, (hetero)aroylamino, (di)C₁₋₂₀ alkylamino(thio) carbonylamino, C₁₋₂₀ alkoxy(thio)carbonylamino, (thio) carboxylic acid (thio)ester and (thio)carboxylic acid amide, with the first proviso that if any one of T₁, T₂, T₃, T₄ and T₅ is oxygen then the correspondingly borne substituent R₁, R₂, R₃, R₄ or R₅ is absent, and with the second proviso that if any one of T₁, T₂, T₃, T₄ and T₅ is nitrogen then the correspondingly borne substituent R₁, R₂, R₃, R₄ or R₅ cannot be cyano, halogen, azido, carboxyl, C₁₋₂₀ alkylthio, C₃₋₁₀ cycloalkylthio, arylthio, arylalkylthio, acylthio or thioheterocyclic,
- S₁, S₂, S₃, S₄ and S₅ are each substituents independently selected from the group consisting of hydrogen, cyano, halogen, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkyl-thio, acylthio, thioheterocyclic, (thio) carboxylic acid (thio)ester and (thio)carboxylic acid amide, with the proviso that if any one of T₁, T₂, T₃, T₄ and T₅ is oxygen or nitrogen then the correspondingly borne substituent S₁, S₂, S₃, S₄ and S₅ is absent,
- if any one of T₁, T₂, T₃, T₄ and T₅ is a carbon atom then each pair of geminal substituents borne by the same carbon atom, i.e. (R₁,S₁), (R₂,S₂), (R₃,S₃), (R₄,S₄) or (R₅,S₅), may independently form a homocyclic or heterocyclic ring, or an alkylidene, cycloalkylidene or arylalkylidene group, or a carbonyl (oxo) or thiocarbonyl (thioxo) group, or an imino, an oximino or a hydrazone group,
- R₆ is selected from the group consisting of hydrogen, C₁₋₂₀ alkyl, C₂₋₂₀ alkenyl, C₂₋₂₀ alkynyl, C₃₋₁₀ cycloalkyl, C₃₋₁₀ cycloalkenyl, aryl, arylalkyl and heteroaryl,
- R₇ and R₈ are each independently selected from the group consisting of hydrogen, C₁₋₂₀ alkyl, C₂₋₂₀ alkenyl, C₂₋₂₀ alkynyl, C₃₋₁₀ cycloalkyl, C₃₋₁₀ cycloalkenyl, aryl, arylalkyl and heteroaryl, or R₇ and R₈ together may form a homocyclic or heterocyclic group, and
- m and n are each integers independently selected from 0, 1 and 2,
- Y is a moiety represented by the general formula 3 wherein:
- U₁, U₂, U₃, U₄ and U₅ are carbon atoms, or alternatively one of U₁, U₂, U₃, U₄, and U₅ is oxygen or nitrogen while the remaining of U₁, U₂, U₃, U₄ and U₅ are carbon atoms, or alternatively two of U₁, U₂, U₃, U₄ and U₅ are either both nitrogen, or oxygen and nitrogen, while the remaining of U₁, U₂, U₃, U₄ and U₅ are carbon atoms, or alternatively two of non adjacent U₁, U₂, U₃, U₄ and U₅ are oxygen while the remaining of U₁, U₂, U₃, U₄ and U₅ are carbon atoms,
- P₁, P₂, P₃, P₄ and P₅ are each substituents independently selected from the group consisting of hydrogen, cyano, halogen, azido, hydroxyl, amino, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, (di) C₁₋₂₀ alkylamino(thio)carbonyloxy, C₁₋₂₀ alkylthio, C₃₋₁₀ cycloalkylthio, arylthio, arylalkylthio, acylthio, thio-heterocyclic, C₁₋₂₀ alkylamino, C₃₋₁₀ cycloalkylamino, C₂₋₂₀ alkenyl-amino, C₃₋₁₀ cycloalkenylamino, arylamino, arylalkylamino, heterocyclic amino, hydroxyalkylamino, mercaptoalkylamino, C₂₋₂₀ alkynylamino, acylamino, thioacylamino, (hetero)aroylamino, (di)C₁₋₂₀ alkylamino(thio)carbonylamino, C₁₋₂₀ alkoxy(thio)carbonylamino, (thio) carboxylic acid (thio)ester and (thio)carboxylic acid amide, with the first proviso that if any one of U₁, U₂, U₃, U₄ and U₅ is oxygen then the correspondingly borne substituent P₁, P₂, P₃, P₄ or P₅ is absent, and with the second proviso that if any one of U₁, U₂, U₃, U₄ and U₅ is nitrogen then the correspondingly borne substituent P₁, P₂, P₃, P₄ or P₅ cannot be cyano, halogen, azido, carboxyl, C₁₋₂₀ alkylthio, C₃₋₁₀ cycloalkylthio, arylthio, arylalkylthio, acylthio or thioheterocyclic,
- Q₁, Q₂, Q₃, Q₄ and Q₅ are each substituents independently selected from the group consisting of hydrogen, cyano, halogen, carboxyl, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, heteroaryl, arylalkyl, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, (thio) carboxylic acid (thio)ester and (thio)carboxylic acid amide, with the proviso that if any one of U₁, U₂, U₃, U₄ and U₅ is oxygen or nitrogen then the correspondingly borne substituent Q₁, Q₂, Q₃, Q₄ or Q₅ is absent,
- if any one of U₁, U₂, U₃, U₄ and U₅ is a carbon atom then a pair of geminal substituents borne by the same carbon atom, i.e. (P₁,Q₁), (P₂,Q₂), (P₃,Q₃), (P₄,Q₄) or (P₅,Q₅) may independently form a homocyclic or heterocyclic ring, or an alkylidene, cycloalkylidene or arylalkylidene group, or a carbonyl (oxo) or thiocarbonyl (thioxo) group, or an imino, an oximino or a hydrazone group,
- L is a linker selected from the group consisting of a covalent bond, C₁₋₂₀ alkyl, C₃₋₁₀ cycloalkyl, C₂₋₂₀ alkenyl, C₃₋₁₀ cycloalkenyl, C₂₋₂₀ alkynyl, heteroalkyl, cycloheteroalkyl, aryl, heteroaryl, arylalkyl and heteroarylalkyl,
- d represents a moiety for the attachment of X and L, which replaces any one of the substituents R₁, R₂, R₃, R₄, R₅, S₁, S₂, S₃, S₄ and S₅,
- e represents a moiety for the attachment of Y and L, which replaces any one of the substituents P₁, P₂, P₃, P₄, P₅, Q₁, Q₂, Q₃, Q₄ and Q₅, and
- each of d and e is a covalent bond, an oxygen atom, a sulfur atom or a divalent radical selected from the group consisting of -N(Z)-, -(C=O)N(Z)-, - SO₂N(Z)-, -S(=O)N(Z)-, -OP(=O)(OW)N(Z)-, -N(Z)C(=O)N(Z)-, -N(Z)C(=O)O-, -N(Z)C(=S)N(Z)-, -N(Z)C(=S)O-, -C(=O)O-, -N(Z)C(=S)Sand -C(=O)-,
- Z is selected from the group consisting of hydrogen, C₁₋₂₀ alkyl, C₂₋₂₀ alkenyl, C₂₋₂₀ alkynyl, aminoalkyl, aminoaryl, aryloxy, C₁₋₂₀ alkoxy, heteroaryloxy, aminoaryl, aminoheteroaryl, thioalkyl, thioaryl, thioheteroaryl, acyl, arylacyl, heteroarylacyl, aryl, heteroaryl, arylalkyl and heteroarylalkyl, and
- W is selected from the group consisting of hydrogen, C₁₋₂₀ alkyl, C₂₋₂₀ alkenyl, C₂₋₂₀ alkynyl, aryl, heteroaryl, arylalkyl and heteroarylalkyl,
a salt, a solvate, a stereoisomer or an enantiomer thereof,
or a monocyclic compound having the general formula 2 as defined herein, a salt, a solvate, a stereoisomer or an enantiomer thereof.

2. A compound according to claim 1, wherein one of A and B is hydrogen and wherein the other of A and B is selected from the group consisting of hydrogen, halogen, azido, C=NOR₆, C=N-NR₇R₈, COOR₆, CONR₇R₈, C₁₋₂₀ alkyl, halo C₁₋₂₀ alkyl, C₃₋₁₀ cycloalkyl, C₃₋₁₀ cycloalkenyl, C₂₋₂₀ alkenyl, C₂₋₂₀ alkynyl, aryl, arylalkyl, alkoxyaryl and heterocyclic, C₁₋₂₀ alkoxy, C₂₋₂₀ alkenyloxy, C₂₋₂₀ alkynyloxy, C₃₋₁₀ cycloalkoxy, C₃₋₁₀ cycloalkenyloxy, aryloxy, arylalkyloxy, acyloxy, oxyheterocyclic, C₁₋₂₀ alkylthio, C₃₋₁₀ cycloalkylthio, arylthio, arylalkylthio, acylthio, thioheterocyclic, C₁₋₂₀ alkylamino, C₃₋₁₀ cycloalkylamino, C₂₋₂₀ alkenylamino, C₃₋₁₀ cycloalkenylamino, arylamino, arylalkylamino, heterocyclic amino, hydroxyalkylamino, mercaptoalkylamino and C₂₋₂₀ alkynylamino, acylamino and thioacylamino.

3. A compound according to claim 1 or claim 2, wherein m is 1 and wherein none of R₂ and S₂ is hydrogen.

4. A compound according to claim 1, wherein each of S₁, S₂, S₃, S₄ and S₅ is hydrogen (i.e. each ring atom of the moiety represented by the general formula **2** is monosubstituted) and further wherein each of Q₁, Q₂, Q₃, Q₄ and Q₅ is hydrogen (i.e. each ring atom of the moiety represented by the general formula **3** is monosubstituted).

5. A compound according to claim 1, wherein no more than two of S₁, S₂, S₃, S₄ and S₅ is hydrogen and/or no more than two of Q₁, Q₂, Q₃, Q₄ and Q₅ is hydrogen.

6. A compound according to any of claims 1 to 5, being in a E stereoisomeric form.

7. A compound according to any of claims 1 to 5, being in a Z stereoisomeric form.

8. A pharmaceutical composition comprising one or more pharmaceutically acceptable carriers and an effective amount of a compound according to any of claims 1 to 7.

9. A pharmaceutical composition according to claim 8, comprising from 0.1 % to 50 % by weight of said compound.
